# EUROPEAN PATENT APPLICATION

(11) **EP 4 703 352 A1**
(43) Date of publication of application: **04.03.2026**
(21) Application number: 24796815.9
(22) Date of filing: 11.04.2024
(51) Int. Cl.: C07C 323/66, C07C 25/00, C07C 25/02, C07C 381/00, C07C 381/02, C07C 381/12, C07D 209/48, C07D 213/71, C07D 221/06, C07D 311/10, C07D 311/12, C07D 401/12, C07D 405/12, C08F 220/38, C09K 3/00, G03F 7/004, G03F 7/039

(54) **SULFONATE, OXIME SULFONATE, IMIDE SULFONATE, AMIDE SULFONATE, ACID GENERATING AGENT CONTAINING SAID COMPOUND, AND PHOTORESIST CONTAINING SAID ACID GENERATING AGENT**

(30) Priority: 26.04.2023 JP 2023072111; 16.06.2023 JP 2023099630
(71) Applicant: San-Apro Ltd., Kyoto-shi, Kyoto 605-0995 (JP)
(72) Inventor: SHIRAISHI, Atsushi, Kyoto-shi, Kyoto 605-0995 (JP)
(74) Representative: Hoffmann Eitle
(86) International application number: PCT/JP2024/014679
(87) International publication number: WO 2024/225059

(57) **Abstract**

Provided are a novel compound that does not have a perfluoromethyl group or a perfluoromethylene group but easily decomposes under light irradiation to generate a sulfonic acid with high acid strength, an acid generator containing the compound, and a photoresist containing the acid generator. The compound of the present invention is a salt of an anion represented by formula (a-1) or (a-2) and a cation. In each formula, R¹ represents an optionally substituted hydrocarbon group or heterocyclic group. L represents a single bond or a linking group. R¹⁰ represents a fluorine atom, an aromatic hydrocarbon group, or an aromatic heterocyclic group, and n represents 0 or 1.

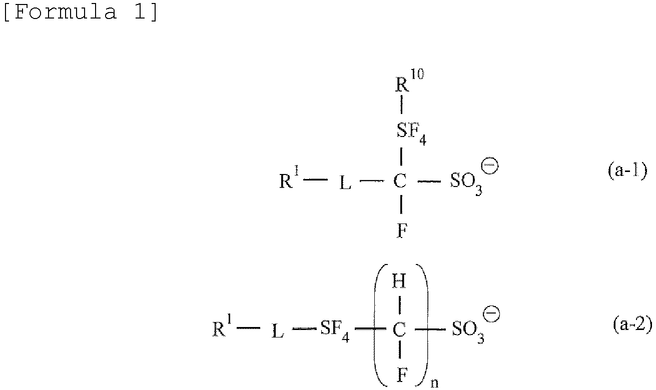

## Description

### Technical Field

The present invention relates to a novel sulfonate, a novel oxime sulfonate, a novel imide sulfonate, a novel amide sulfonate, an acid generator containing the novel compound, and a photoresist containing the acid generator.

### Background Art

Compounds that decompose under irradiation with light to generate acids (so-called acid generators) are used in various fields, for example, in the field of semiconductor resists, to alter the solubility of a chemically amplified resist used in photolithography in a developing solution. The compounds are also used in the field of paints, adhesives, modeling materials for various parts, sealant, and the like, to initiate cationic polymerization of a cationically curable compound for curing.

As the acid generators, compounds represented by formulae (X-1) and (X-2) below are known (see Patent Literatures 1 and 2). These compounds have a perfluoromethyl group or a perfluoromethylene group at the α-position of the sulfonic acid group, allowing for generation of a sulfonic acid with high acid strength.

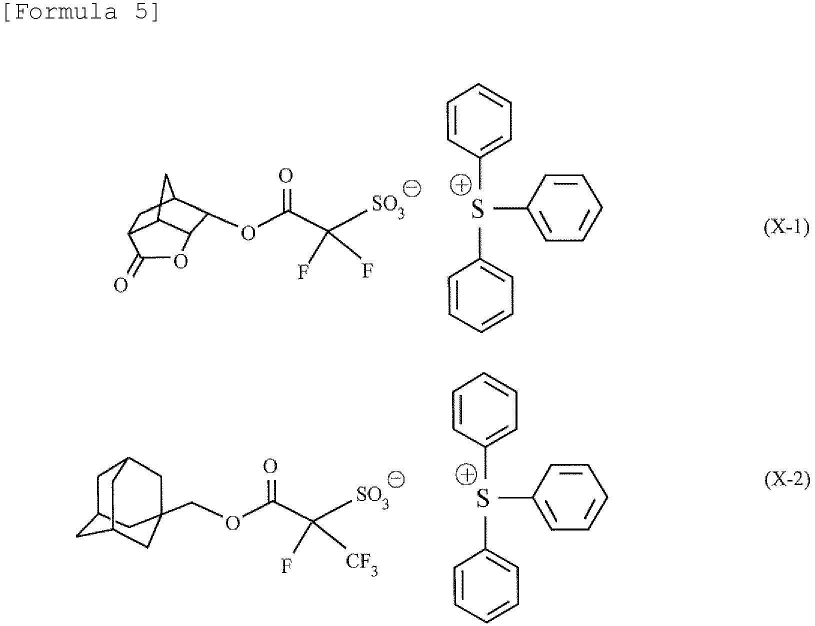

### Citation List

### Patent Literature

Patent Literature 1: Japanese Patent Laid-Open No. 2006-306856
Patent Literature 2: Japanese Patent Laid-Open No. 2008-297255

### Summary of Invention

### Technical Problem

However, in Europe, the use of compounds having a perfluoromethyl group or a perfluoromethylene group, such as the compounds represented by formulae (X-1) and (X-2), is restricted due to the tightening of Per and Polyfluoroalkyl Substances (PFAS) regulations, and substitutes are thus required.

Therefore, an object of the present invention is to provide a novel compound that does not have a perfluoromethyl group or a perfluoromethylene group, but easily decomposes under light irradiation to generate a sulfonic acid with high acid strength.

Another object of the present application is to provide an acid generator containing the novel compound.

Another object of the present application is to provide a photoresist containing the acid generator.

Another object of the present application is to provide a method for manufacturing an electronic device or an optical device using the photoresist.

### Solution to Problem

As a result of intensive studies to achieve the objects, the present inventors have found that a compound which is a salt of an anion represented by formula (a-1) or (a-2) and a cation, a compound represented by formula (b-1) or (b-2), a compound represented by formula (c-1) or (c-2), and a compound represented by formula (d-1) or (d-2) all do not have a perfluoromethyl group or a perfluoromethylene group at the α-position of the sulfonic acid group, but decompose under light irradiation to generate a sulfonic acid with high acid strength. On the basis of these findings, the present invention has been completed.

That is, the present invention provides a compound which is a salt of an anion represented by formula (a-1) or (a-2): 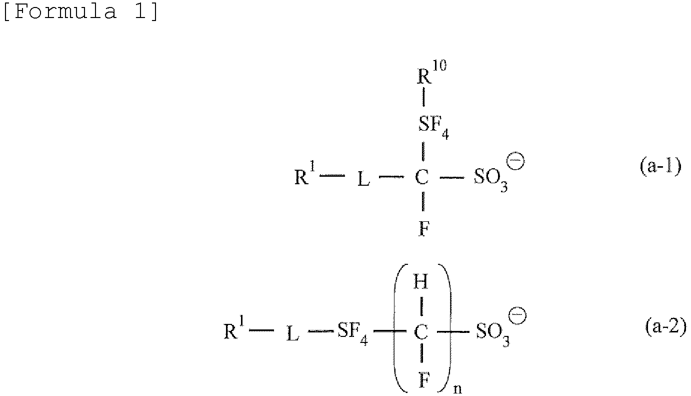 and a cation, wherein R¹ represents an optionally substituted hydrocarbon group or heterocyclic group; L represents a single bond or a linking group; and R¹⁰ represents a fluorine atom, an aromatic hydrocarbon group, or an aromatic heterocyclic group, and n represents 0 or 1.

The present invention provides a compound represented by formula (b-1) or (b-2): 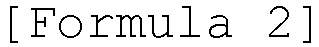 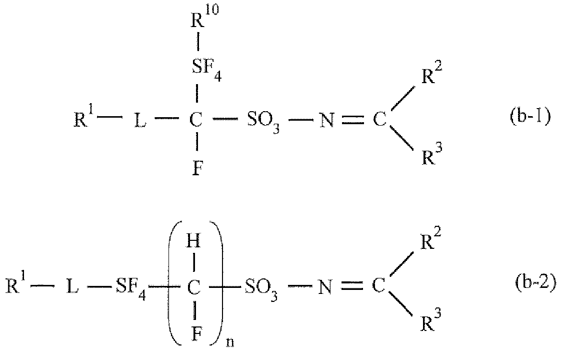 wherein R¹ represents an optionally substituted hydrocarbon group or heterocyclic group; L represents a single bond or a linking group; R² and R³ are the same as or different from each other and each represent an organic group; and R¹⁰ represents a fluorine atom, an aromatic hydrocarbon group, or an aromatic heterocyclic group, and n represents 0 or 1.

The present invention provides a compound represented by formula (c-1) or (c-2): 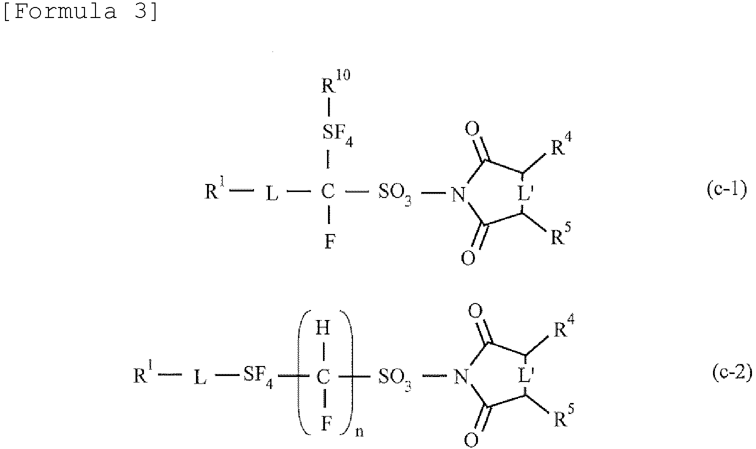 wherein R¹ represents an optionally substituted hydrocarbon group or heterocyclic group; L and L' are the same as or different from each other and each represents a single bond or a linking group. R⁴ and R⁵ are the same as or different from each other and each represent an optionally substituted hydrocarbon group; R⁴ and R⁵ are optionally linked to each other to form a ring together with an adjacent carbon atom; and R¹⁰ represents a fluorine atom, an aromatic hydrocarbon group, or an aromatic heterocyclic group, and n represents 0 or 1.

The present invention provides a compound represented by formula (d-1) or (d-2): 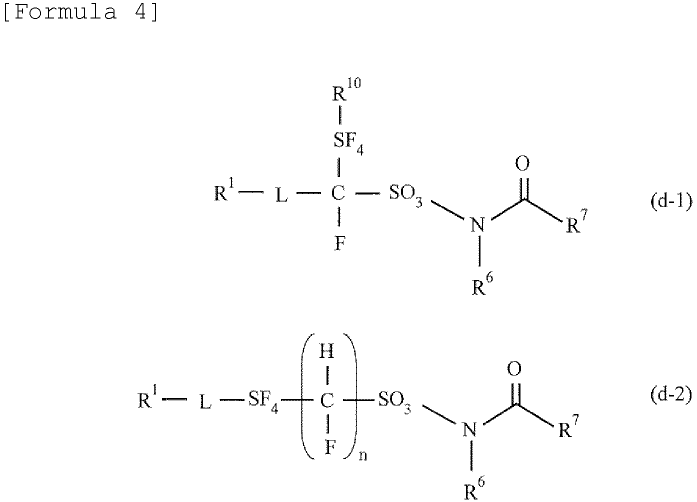 wherein R¹ represents an optionally substituted hydrocarbon group or heterocyclic group; L represents a single bond or a linking group; R⁶ and R⁷ are the same as or different from each other and each represent an optionally substituted hydrocarbon group; and R¹⁰ represents a fluorine atom, an aromatic hydrocarbon group, or an aromatic heterocyclic group, and n represents 0 or 1.

The present invention provides an acid generator containing the compound.

The present invention also provides a photoresist containing the acid generator and an acid-reactive compound.

In addition, the present invention provides a method for manufacturing an electronic device or an optical device, comprising the step of forming a pattern by photolithography using the photoresist.

### Advantageous Effects of Invention

The compound of the present invention includes a compound which is a salt of an anion represented by formula (a-1) or (a-2) and a cation, a compound represented by formula (b-1) or (b-2), a compound represented by formula (c-1) or (c-2), and a compound represented by formula (d-1) or (d-2), and rapidly decomposes upon irradiation with light to generate a sulfonic acid represented by (A-1) or (A-2) described below.

The compound of the present invention can generate a sulfonic acid with an acid strength equal to or higher than that of an acid generated by a conventional acid generator having a perfluoromethyl group and a perfluoromethylene group.

A resist film having a high-resolution pattern with good accuracy can be manufactured by using a photoresist containing the compound having the above characteristics. When a substrate is subjected to an etching (for example, dry etching using a reactive gas or plasma) process using the resist film obtained, a semiconductor device having a high-resolution pattern (for example, a wiring pattern or a circuit pattern) can be manufactured with a high yield.

### Description of Embodiments

### Sulfonate

A sulfonate of the present invention is a compound which is a salt of an anion represented by formula (a-1) or (a-2): 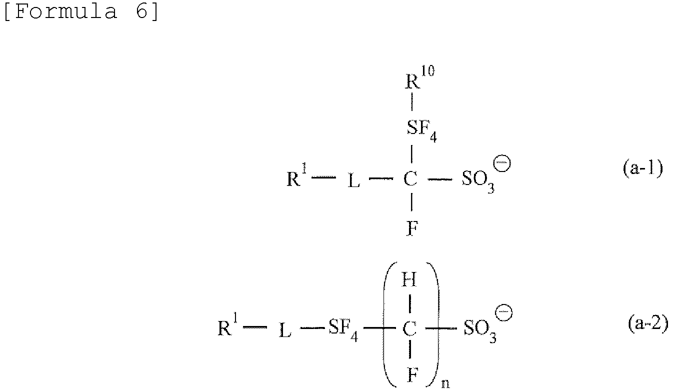 and a cation, wherein R¹ represents an optionally substituted hydrocarbon group or heterocyclic group; L represents a single bond or a linking group; and R¹⁰ represents a fluorine atom, an aromatic hydrocarbon group, or an aromatic heterocyclic group, and n represents 0 or 1.

The linking group in L is a divalent group having one or more atoms, and examples thereof include a divalent hydrocarbon group, a divalent heterocyclic group, a carbonyl group (-CO-), an ether bond (-O-), a thioether bond (-S-), a sulfonyl group (-SO₂-), an ester bond (-COO- or -OCO-), an amide bond (-CONH-), a carbonate bond (-OCOO-), and a group in which a plurality of these groups are linked.

The divalent hydrocarbon group or divalent heterocyclic group may have an electron-withdrawing group as a substituent (for example, a carboxyl group, a C₁₋₅ alkyl ester group, a nitro group, a halogen atom, a C₁₋₁₄ acyl group, a cyano group, a tosyl group, or a mesyl group). The divalent hydrocarbon group and the divalent heterocyclic group will be described in detail later.

In formula (a-1), L is preferably a group containing at least an ester bond (-COO- or -OCO-).

That is, the anion represented by the formula (a-1) is preferably an anion represented by formula (a-1-1) or (a-1-2): 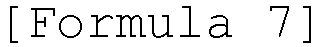 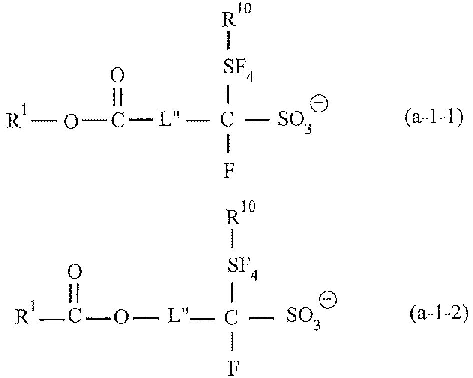 wherein R¹ represents an optionally substituted hydrocarbon group or heterocyclic group; L" represents a single bond or a linking group; and R¹⁰ represents a fluorine atom, an aromatic hydrocarbon group, or an aromatic heterocyclic group, and n represents 0 or 1.

Examples of the linking group in L" include the divalent groups listed as linking groups in L above, other than ester bonds (-COO- and -OCO-). In particular, L" is preferably a single bond, a divalent hydrocarbon group, or a divalent heterocyclic group.

R¹ represents an optionally substituted (monovalent) hydrocarbon group or (monovalent) heterocyclic group.

Examples of the monovalent hydrocarbon group include a monovalent aliphatic hydrocarbon group, a monovalent alicyclic hydrocarbon group, a monovalent aromatic hydrocarbon group, and a monovalent group formed by bonding these groups.

The monovalent aliphatic hydrocarbon group is preferably a C₁₋₂₀ aliphatic hydrocarbon group. The monovalent aliphatic hydrocarbon group includes a monovalent saturated aliphatic hydrocarbon group and a monovalent unsaturated aliphatic hydrocarbon group. The monovalent saturated aliphatic hydrocarbon group is preferably a C₁₋₂₀ saturated aliphatic hydrocarbon group, and examples thereof include alkyl groups having about 1 to 20 (preferably 1 to 10, particularly preferably 1 to 3) carbon atoms, such as a methyl group, an ethyl group, a propyl group, an isopropyl group, a butyl group, an isobutyl group, a s-butyl group, a t-butyl group, a pentyl group, a hexyl group, a decyl group, and a dodecyl group. The monovalent unsaturated aliphatic hydrocarbon group is preferably a C₂₋₂₀ unsaturated aliphatic hydrocarbon group, and examples thereof include alkenyl groups having about 2 to 20 (preferably 2 to 10, particularly preferably 2 to 3) carbon atoms, such as a vinyl group, an allyl group, and a 1-butenyl group; and alkynyl groups having about 2 to 20 (preferably 2 to 10, particularly preferably 2 or 3) carbon atoms, such as an ethynyl group and a propynyl group.

The monovalent alicyclic hydrocarbon group is preferably a C₃₋₂₀ alicyclic hydrocarbon group, and examples thereof include 3- to 20-membered (preferably 3-to 15-membered, particularly preferably 5- to 8-membered) cycloalkyl groups, such as a cyclopropyl group, a cyclobutyl group, a cyclopentyl group, a cyclohexyl group, and a cyclooctyl group; 3- to 20-membered (preferably 3- to 15-membered, particularly preferably 5-to 8-membered) cycloalkenyl groups, such as a cyclopentenyl group and a cyclohexenyl group; and bridged cyclic hydrocarbon groups, such as a perhydronaphthalen-1-yl group, a norbornyl group, an adamantyl group, a tricyclo[5.2.1.0^{2,6}]dec-8-yl group, and a tetracyclo [4.4.0.1^{2,5}.1^{7,10}] dodec-3-yl group.

The monovalent aromatic hydrocarbon group is preferably a C₆₋₁₄ (especially C₆₋₁₀) aromatic hydrocarbon group, and examples thereof include a phenyl group and a naphthyl group.

Examples of a monovalent group formed by bonding of an alicyclic hydrocarbon group and an aromatic hydrocarbon group include C₇₋₁₈ aralkyl groups such as a benzyl group and a phenylethyl group.

In addition, a ring of the alicyclic hydrocarbon group or aromatic hydrocarbon group may be fused with an aromatic or non-aromatic heterocyclic ring.

Examples of the substituents that the hydrocarbon group may have include halogen atoms, an oxo group, a hydroxy group, substituted oxy groups (for example, C₁₋₄ alkoxy groups, C₆₋₁₀ aryloxy groups, C₇₋₁₆ aralkyloxy groups, or C₁₋₄ acyloxy groups), a thio group, substituted thio groups (for example, C₁₋₆ alkylthio groups or C₆₋₁₀ arylthio groups, which may have substituents, examples of which include a hydroxy group, a cyano group, an alkoxy group, a hydroxyalkoxy group, a sulfo group, and a carboxyl group), a carboxyl group, substituted oxycarbonyl groups (for example, C₁₋₄ alkoxycarbonyl groups, C₆₋₁₀ aryloxycarbonyl groups, and C₇₋₁₆ aralkyloxycarbonyl groups), acyl groups (including C₁₋₆ aliphatic acyl groups such as a formyl group, an acetyl group, a propionyl group, a butyryl group, an isobutyryl group, and a pivaloyl group; acetoacetyl groups; and C₇₋₁₄ aromatic acyl groups such as a benzoyl group), substituted or unsubstituted carbamoyl groups (for example, a carbamoyl, C₁₋₄ alkyl-substituted carbamoyl such as methylcarbamoyl, and C₆₋₁₀ aryl-substituted carbamoyl groups such as a phenylcarbamoyl group), a cyano group, a nitro group, a sulfo group, and heterocyclic groups. Additionally, the hydrocarbon group may have a group including a perfluoromethyl group or a perfluoromethylene group (for example, a C₁₋₁₀ haloalkyl group).

The monovalent heterocyclic group is a group derived from the structural formula of a heterocyclic ring by removing one hydrogen atom. The heterocyclic ring includes an aromatic heterocyclic ring and a non-aromatic heterocyclic ring. The heterocyclic ring constituting the heterocyclic group includes an aromatic heterocyclic ring and a non-aromatic heterocyclic ring. Examples of such a heterocyclic ring include 3- to 20-membered rings (preferably 3- to 10-membered rings, particularly preferably 4- to 6-membered rings) having a carbon atom and at least one heteroatom (for example, an oxygen atom, a sulfur atom, or a nitrogen atom) as atoms constituting the ring, and a fused ring thereof. Specific examples thereof include heterocyclic rings containing an oxygen atom as a heteroatom (for example, 3-membered rings such as an oxirane ring; 4-membered rings such as an oxetane ring; 5-membered rings such as a furan ring, a tetrahydrofuran ring, an oxazole ring, an isoxazole ring, and a γ-butyrolactone ring; 6-membered rings such as a 4-oxo-4H-pyran ring, a tetrahydropyran ring, and a morpholine ring; fused rings such as a benzofuran ring, an isobenzofuran ring, a 4-oxo-4H-chromene ring, a chroman ring, and an isochroman ring; bridged rings such as a 3-oxatricyclo[4.3.1.1^{4,8}]undecan-2-one ring and a 3-oxatricyclo[4.2.1.0^{4,8}]nonan-2-one ring), heterocyclic rings containing a sulfur atom as a heteroatom (for example, 5-membered rings such as a thiophene ring, a thiazole ring, an isothiazole ring, and a thiadiazole ring; 6-membered rings such as a 4-oxo-4H-thiopyran ring; fused rings such as a benzothiophene ring), and heterocyclic rings containing a nitrogen atom as a heteroatom (for example, 5-membered rings such as a pyrrole ring, a pyrrolidine ring, a pyrazole ring, an imidazole ring, and a triazole ring; 6-membered rings such as an isocyanurate ring, a pyridine ring, a pyridazine ring, a pyrimidine ring, a pyrazine ring, a piperidine ring, and a piperazine ring; fused rings such as an indole ring, an indoline ring, a quinoline ring, an acridine ring, a naphthyridine ring, a quinazoline ring, and a purine ring).

Examples of the substituents that the heterocyclic group may have, in addition to the substituents that the hydrocarbon group may have, include an alkyl group (for example, a C₁₋₄ alkyl group such as a methyl group and an ethyl group), an alkenyl group (for example, a C₂₋₄ alkenyl group such as a vinyl group, an allyl group, and a 1-butenyl group), an alkynyl group (for example, a C₂₋₄ alkynyl group such as an ethynyl group and a propynyl group), a C₃₋₈ cycloalkyl group, and an aryl group (for example, a C₆₋₁₅ aryl group such as a phenyl group, a naphthyl group, and a biphenyl group).

R¹ is preferably a group having a molecular weight of 100 or more (for example, 100 to 200, preferably 100 to 150) from the viewpoint of suppressing the diffusivity of a sulfonic acid to be generated and producing an effect of improving the pattern resolution when used in a photoresist.

Examples of the divalent hydrocarbon group include groups derived from the structural formula of the monovalent hydrocarbon group by removing one hydrogen atom. Preferred examples thereof include C₁₋₅ alkylene groups such as a methylene group, a methylmethylene group, a dimethylmethylene group, an ethylene group, a propylene group, and a trimethylene group; C₃₋₆ cycloalkylene groups such as a cyclopentylene group and a cyclohexylene group; and C₆-₁₀ arylene groups such as a phenylene group, a phenylenebis(methylene) group, a biphenylene group, and a naphthylene group.

Examples of the divalent heterocyclic group include groups derived from the structural formula of the heterocyclic ring by removing two hydrogen atoms. Preferred examples thereof include groups derived from the structural formula of a heterocyclic ring containing a nitrogen atom as a hetero atom, such as a pyridylene group and a quinolylene group.

R¹⁰ represents a fluorine atom, an aromatic hydrocarbon group, or an aromatic heterocyclic group.

The aromatic hydrocarbon group in R¹⁰ is a monovalent aromatic hydrocarbon group, preferably a C₆₋₁₄ (particularly, C₆₋₁₀) aromatic hydrocarbon group, for example, a phenyl group or a naphthyl group. The aromatic hydrocarbon group may have substituents. Examples of the substituents are the same as the substituents that the hydrocarbon group may have (for example, a halogen atom).

The aromatic heterocyclic group in R¹⁰ is a monovalent aromatic heterocyclic group derived from the structural formula of an aromatic heterocyclic ring by removing one hydrogen atom. Examples of the aromatic heterocyclic ring include 5-membered rings such as a pyrrole ring, an imidazole ring, a pyrazole ring, a triazole ring, a furan ring, and a thiophene ring; 6-membered rings such as a pyridine ring, a pyrazine ring, a pyrimidine ring, and a pyridazine ring; and fused rings such as a quinoline ring and a benzofuran ring.

Specifically, R¹⁰ is preferably a fluorine atom or an aromatic hydrocarbon group, particularly preferably a fluorine atom in order to provide excellent photosensitivity and generate a sulfonic acid with high acid strength, thereby producing an effect of improving the resolution of the photoresist.

n represents 0 or 1.

Specifically, n is preferably 0 in order to provide excellent photosensitivity and generate a sulfonic acid with high acid strength, thereby producing an effect of improving the resolution of the photoresist.

Therefore, the anion represented by formula (a-1) is preferably an anion represented by formula (a-1'), particularly preferably an anion represented by formula (a-1'-1) or (a-1'-2). In the following formula, R¹, L, and L" are as defined above.

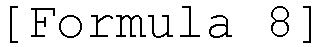

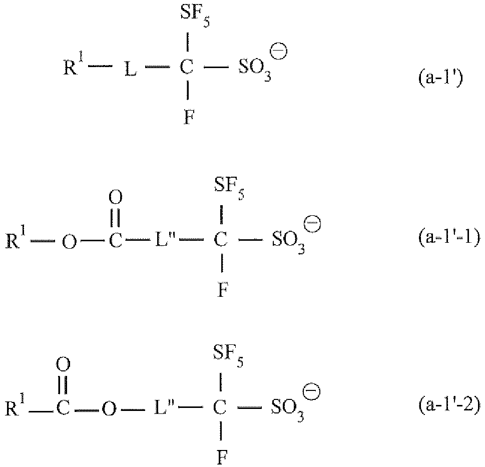

The anion represented by formula (a-1) is preferably an anion represented by formula (a-1"), particularly preferably an anion represented by formula (a-1"-1) or (a-1"-2). In the following formula, L and L" are as defined above.

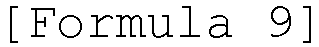

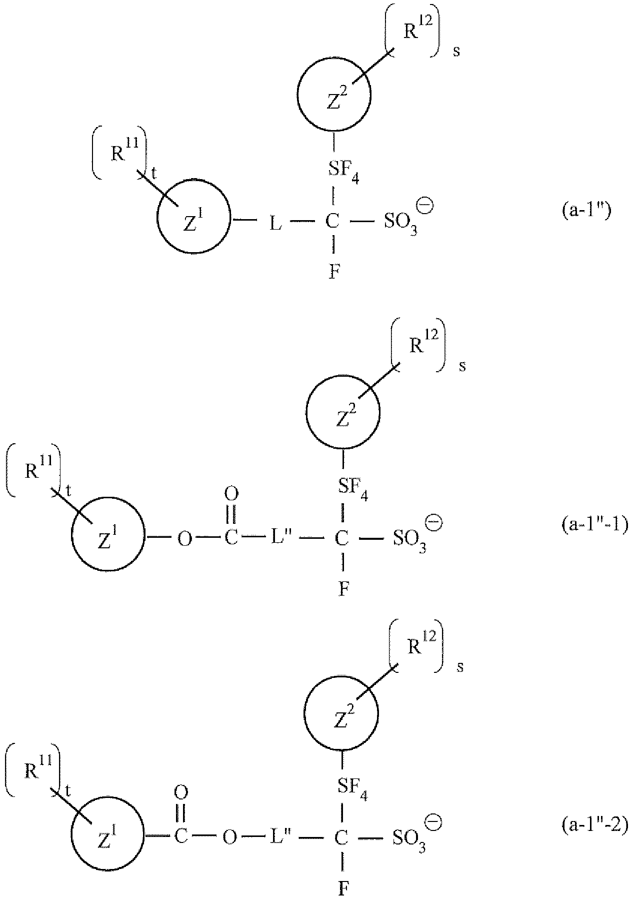

In the formula, a ring Z¹ represents an alicyclic hydrocarbon, an aromatic hydrocarbon ring, or an aromatic heterocyclic ring. A ring Z² represents an aromatic hydrocarbon ring or an aromatic heterocyclic ring.

Specifically, the ring Z¹ is preferably an alicyclic hydrocarbon, particularly preferably a C₃₋₂₀ alicyclic hydrocarbon group.

Specifically, the ring Z² is preferably an aromatic hydrocarbon ring, particularly preferably a benzene ring.

R¹¹ and R¹² are substituents attached to the rings Z¹ and Z², respectively. Examples of the substituents are the same as the substituents that the hydrocarbon group and the heterocyclic group in R¹ and R¹⁰ may have. Specifically, the substituents are preferably halogen atoms. S and t are the same as or different from each other, and each represent an integer of 0 to 5. Specifically, s is preferably 0 or 1, particularly preferably 1. Specifically, t is preferably 0 or 1, particularly preferably 0.

The anion represented by formula (a-2) is preferably an anion represented by formula (a-2'), particularly preferably an anion represented by formula (a-2'-1). In the following formula, R¹, L, and L" are as defined above.

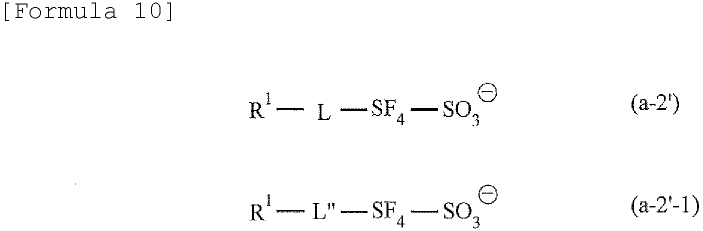

The anion represented by formula (a-2) is preferably an anion represented by formula (a-2"), particularly preferably an anion represented by formula (a-2"-1). In the following formula, L is defined as above.

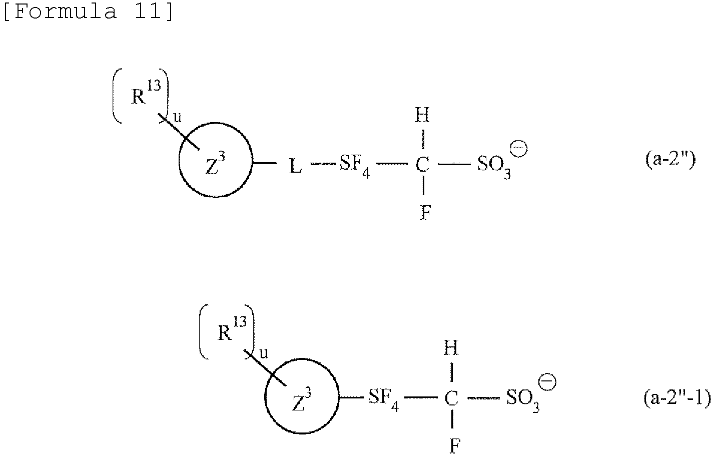

In the formula, a ring Z³ represents an alicyclic hydrocarbon, an aromatic hydrocarbon ring, or an aromatic heterocyclic ring. Specifically, the ring Z³ is preferably an aromatic hydrocarbon ring or an aromatic heterocyclic ring, particularly preferably an aromatic hydrocarbon ring.

R¹³ is a substituent attached to the ring Z³. Examples of the substituent are the same as the substituent that the hydrocarbon group and the heterocyclic group in R¹ and R¹⁰ may have. Specifically, the substituent is preferably a halogen atom, a nitro group, or a C₁₋₄ acyl oxy group. u represents an integer from 0 to 5. Specifically, u is preferably 0 or 1, particularly preferably 1.

Cations constituting the sulfonate are not particularly limited, and may be monovalent cations or polyvalent cations of divalent or higher valence. Specifically, the cations in the present invention are preferably monovalent or divalent cations, particularly preferably monovalent cations.

The cations include an inorganic cation and an organic cation.

In the case of a salt of the anion represented by formula (a-1) or (a-2) and an inorganic cation, the sulfonate is referred to as an inorganic sulfonate.

The inorganic sulfonate can be suitably used, for example, as a raw material for an organic sulfonate.

In the case of a salt of the anion represented by formula (a-1) or (a-2) and an organic cation, the sulfonate is referred to as an organic sulfonate.

The organic sulfonate can be suitably used as an acid generator.

Examples of the inorganic cation include alkali metal ions such as a sodium ion, a potassium ion, and a lithium ion; and alkaline earth metal ions such as a calcium ion, a magnesium ion, and a barium ion.

Examples of the organic cation include onium cations such as a sulfonium ion, an iodonium ion, a selenium ion, an ammonium ion, and a phosphonium ion.

Examples of the sulfonium ion include a sulfonium ion represented by formula (s).

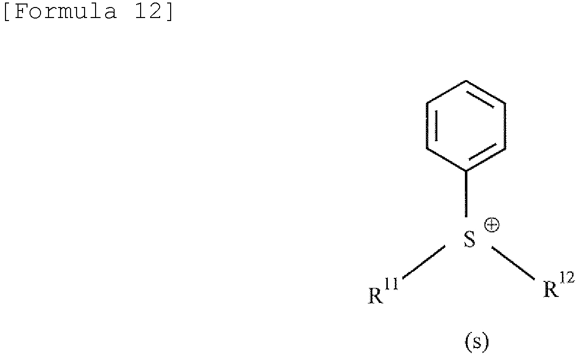

In the formula, R¹¹ and R¹² are the same as or different from each other, and each represent a monovalent hydrocarbon group, a monovalent heterocyclic group, and a monovalent group formed by bonding two or more of the groups via a single bond or a linking group. R¹¹ and R¹² are optionally linked to each other to form a ring together with an adjacent S⁺.

Examples of the monovalent hydrocarbon group and the monovalent heterocyclic group in R¹¹ and R¹² are the same as the hydrocarbon group and the heterocyclic group in R¹. The group may have substituents, and examples of the substituents are the same as the substituent that the hydrocarbon group and heterocyclic group in R¹ may have.

The ring that may be formed by R¹¹ and R¹² linked to each other, together with adjacent S⁺, is a heterocyclic ring containing at least S⁺ described in the formula. The heterocyclic ring may contain a heteroatom (for example, an oxygen atom, a nitrogen atom, or a sulfur atom) in addition to S⁺. The heterocyclic ring is, for example, a 5- or 6-membered heterocyclic ring, and includes an aromatic heterocyclic ring and a non-aromatic heterocyclic ring. The heterocyclic ring may be fused with an aromatic hydrocarbon ring.

An alkyl group (for example, a C₁₋₅ alkyl group) or an aryl group (for example, a C₆₋₁₅ aryl group) may be bonded to the benzene ring represented in the formula. An aromatic hydrocarbon ring may be fused to the benzene ring. Furthermore, substituents may be bonded to the benzene ring, an alkyl group or aryl group bonded to the benzene ring, or a fused ring of the benzene ring and the aromatic hydrocarbon ring. Examples of the substituents include a hydroxy group, a SF₅ group, a halogen atom, a silyl group, a substituted oxy group (for example, a C₁₋₄ alkoxy group, a C₆₋₁₅ aryloxy group, a C₇₋₁₆ aralkyloxy group, or a C₁₋₄ acyloxy group), and a substituted thio group (for example, a C₁₋₆ alkylthio group or a C₆₋₁₅ arylthio group).

The sulfonium ion may be bonded to an acid-reactive compound described later, that is, the sulfonium ion may contain an acid-reactive compound described later in its structure.

Specifically, the sulfonium ion represented by formula (s) is preferably a triarylsulfonium ion represented by formula (s-1) or a sulfonium ion represented by formula (s-2).

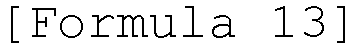

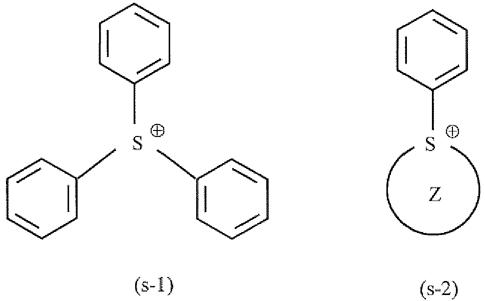

A ring Z in formula (s-2) is a heterocyclic ring containing at least a sulfur atom as a heteroatom. The heterocyclic ring may contain a heteroatom (for example, an oxygen atom, a nitrogen atom or a sulfur atom) in addition to the sulfur atom. The heterocyclic ring is, for example, a 5- or 6-membered heterocyclic ring, and includes an aromatic heterocyclic ring and a non-aromatic heterocyclic ring. The heterocyclic ring may be fused with an aromatic hydrocarbon ring.

Specific examples of the sulfonium ion represented by formula (s-2) are shown below. The same substituents as described above may also be bonded to the benzene ring represented by the following formula.

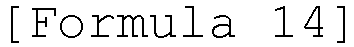

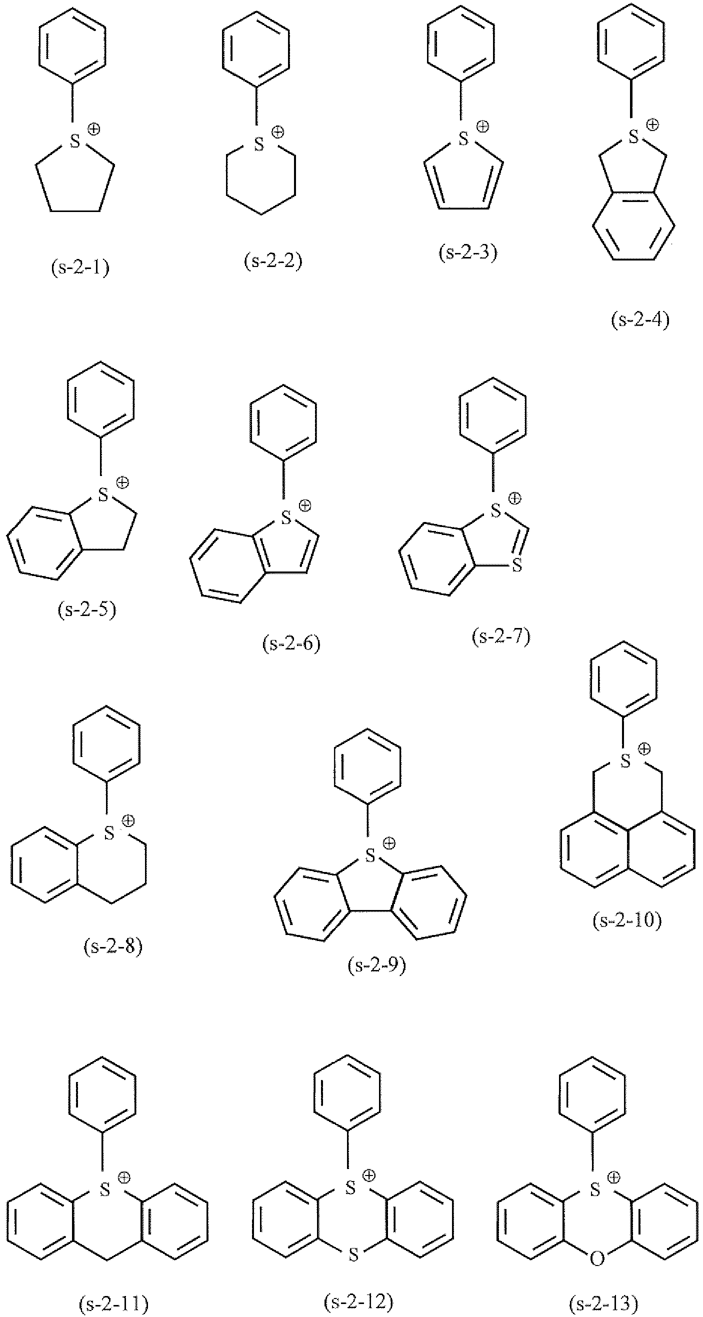

Examples of the iodonium ion include an iodonium ion represented by formula (i).

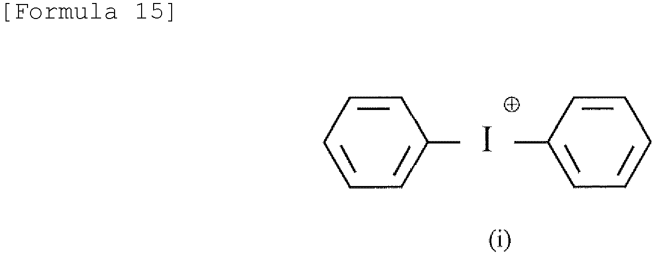

An alkyl group (for example, a C₁₋₅ alkyl group) or an aryl group (for example, a C₆₋₁₅ aryl group) may be bonded to the benzene ring represented in the formula. An aromatic hydrocarbon ring may be fused to the benzene ring. Furthermore, substituents may be bonded to the benzene ring, an alkyl group or aryl group bonded to the benzene ring, or a fused ring of the benzene ring and the aromatic hydrocarbon ring. Examples of the substituents include a hydroxy group, a SF₅ group, a halogen atom, a silyl group, a substituted oxy group (for example, a C₁₋₄ alkoxy group, a C₆₋₁₅ aryloxy group, a C₇₋₁₆ aralkyloxy group, or a C₁₋₄ acyloxy group), and a substituted thio group (for example, a C₁₋₆ alkylthio group or a C₆₋₁₅ arylthio group).

Specific examples of the iodonium ion include aryliodonium ions such as a p-cumenyl(p-tolyl)iodonium ion, a diphenyliodonium ion, a di-p-tolyliodonium ion, a bis(4-tert-butylphenyl)iodonium ion, a bis(4-dodecylphenyl)iodonium ion, a bis(4-methoxyphenyl)iodonium ion, and a (4-octyloxyphenyl)phenyliodonium ion.

Examples of the selenium ion include arylselenium ions such as a triphenylselenium ion, a tri-p-tolylselenium ion, a tri-o-tolylselenium ion, a tris(4-methoxyphenyl)selenium ion, a 1-naphthyldiphenylselenium ion, a tris(4-fluorophenyl)selenium ion, a tri-1-naphthylselenium ion, and a tri-2-naphthylselenium ion.

Examples of the ammonium ion include quaternary ammonium ions such as a tetramethylammonium ion, a tetraethylammonium ion, a tetrapropylammonium ion, a tetrabutylammonium ion, a tetrapentylammonium ion, a tetrahexylammonium ion, a tetraheptylammonium ion, a tetraoctylammonium ion, a trimethylhexylammonium ion, and a trimethyloctylammonium ion.

Examples of the phosphonium ion include tetraarylphosphonium such as tetraphenylphosphonium, tetra-p-tolylphosphonium, tetrakis(2-methoxyphenyl)phosphonium, tetrakis(3-methoxyphenyl)phosphonium, and tetrakis(4-methoxyphenyl)phosphonium; triarylphosphonium such as triphenylbenzylphosphonium, triphenylphenacylphosphonium, triphenylmethylphosphonium, and triphenylbutylphosphonium; and tetraalkylphosphonium such as triethylbenzylphosphonium, tributylbenzylphosphonium, tetraethylphosphonium, tetrabutylphosphonium, tetrahexylphosphonium, triethylphenacylphosphonium, and tributylphenacylphosphonium.

The organic cation is preferably a sulfonium ion or an iodonium ion, particularly preferably an arylsulfonium ion or an aryliodonium ion, from the viewpoint of providing excellent photosensitivity.

The sulfonate rapidly decomposes upon photoirradiation to generate a sulfonic acid.

When the sulfonate contains the anion represented by formula (a-1), a sulfonic acid represented by formula (A-1) is generated.

When the sulfonate contains the anion represented by formula (a-2), a sulfonic acid represented by formula (A-2) is generated.

The sulfonic acid has a SF₄R¹⁰ group or a SF₄ group and thus exhibits high acid strength. 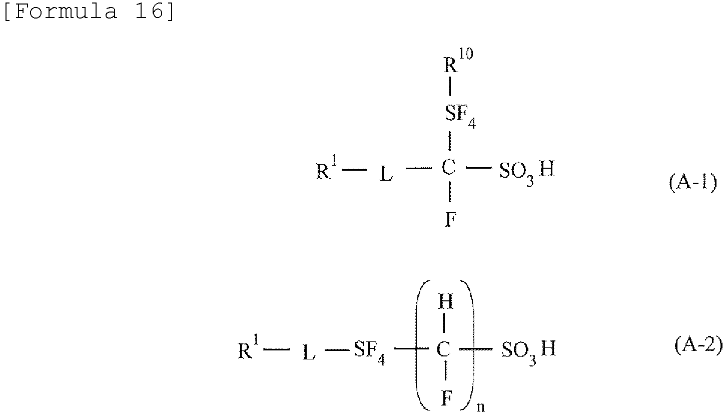 (In the formula, R¹, L, R¹⁰ and n are as defined above.)

The gas phase acidity ΔG (kcal/mol) of the sulfonic acid is, for example, 300 or less, preferably 290 or less, particularly preferably 285 or less, especially preferably 280 or less, and most preferably 275 or less. The lower limit of the gas phase acidity ΔG (Kcal/mol) is, for example, 250, specifically 255, and particularly 260. Herein, the "gas phase acidity" refers to acidity in a gas phase, which is defined as a change in Gibbs energy accompanying acid dissociation by the International Union of Pure and Applied Chemistry (IUPAC). The gas phase acidity can be calculated using known calculation software. The smaller the gas phase acidity, the greater the acid strength.

### Oxime Sulfonate

An oxime sulfonate of the present invention is a compound represented by formula (b-1) or (b-2): 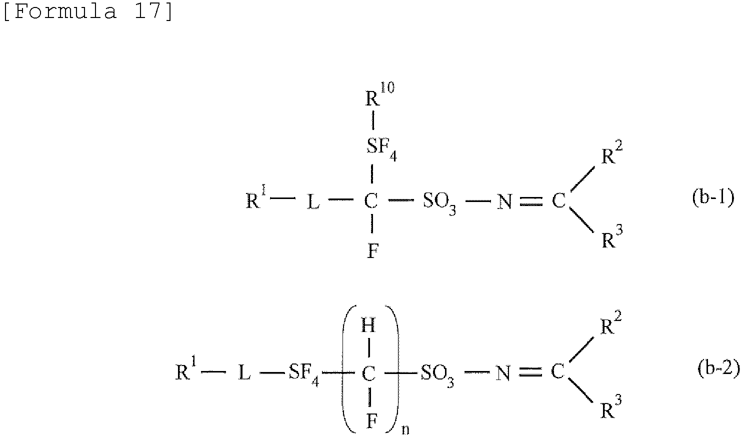 wherein R¹ represents an optionally substituted hydrocarbon group or heterocyclic group; L represents a single bond or a linking group; R² and R³ are the same as or different from each other and each represent an organic group; and R¹⁰ represents a fluorine atom, an aromatic hydrocarbon group, or an aromatic heterocyclic group, and n represents 0 or 1.

The oxime sulfonate rapidly decomposes upon photoirradiation to generate a sulfonic acid.

When the oxime sulfonate is a compound represented by formula (b-1), a sulfonic acid represented by formula (A-1) is generated.

When the oxime sulfonate is a compound represented by formula (b-2), a sulfonic acid represented by formula (A-2) is generated.

The sulfonic acid has a SF₄R¹⁰ group or a SF₄ group and thus exhibits high acid strength.

In formulae (b-1) and (b-2), R¹ and L are the same as R¹ and L in formulae (a-1) and (a-2).

Examples of the organic group in R² and R³ include a hydrocarbon group (R), an RO group, an RCO group, a ROCO group, and an RS group (where each R represents a hydrocarbon group), a carboxyl group, a cyano group, an isocyanato group, a carbamoyl group, an isothiocyanato group, a substituted amino group, a heterocyclic group, and a group formed by bonding two or more of the groups via a single bond.

The organic group in R² and R³ may be a group in which two or more hydrocarbon groups (R) are bonded via a divalent group represented by formula (L-1): 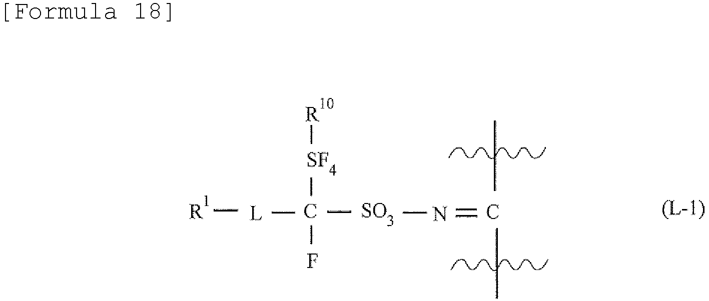 wherein R¹ and L are as defined above; a bond marked with a wavy line in the formula is bonded to a hydrocarbon group in R² or R³; and R¹⁰ represents a fluorine atom, an aromatic hydrocarbon group, or an aromatic heterocyclic group, and n represents 0 or 1.

Examples of the hydrocarbon group (R) and heterocyclic group are the same as those of the hydrocarbon group and heterocyclic group in R¹, respectively. The hydrocarbon group in R² and R³ may have substituents, and examples of the substituents are the same as those of the substituents that the hydrocarbon group in R¹ may have.

One of R² and R³ is preferably a hydrocarbon group that may have substituents. The other of R² and R³ is preferably an electron-withdrawing group, particularly preferably a group selected from a cyano group, an RCO group (where R represents a hydrocarbon group), an isocyanate group, a carbamoyl group, and an isothiocyanate group, and especially preferably a cyano group.

Specifically, the compound represented by formula (b-1) or (b-2) is preferably a compound represented by formula (b-1') or (b-2') in order to provide excellent photosensitivity and generate a sulfonic acid with high acid strength, thereby producing an effect of improving the resolution of the photoresist. In the following formula, R¹, L, R², and R³ are as defined above.

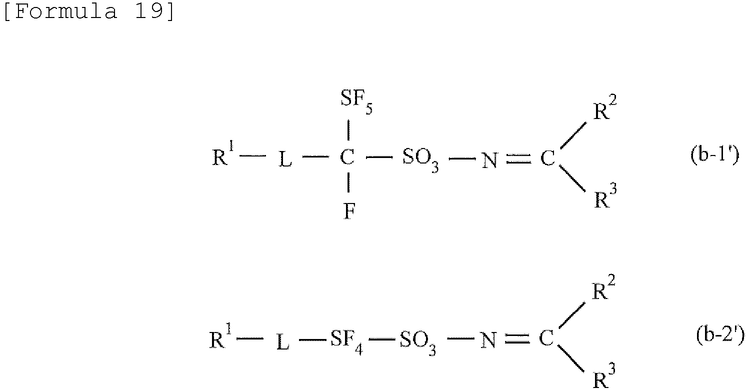

### Imide Sulfonate

An imide sulfonate of the present invention is a compound represented by formula (c-1) or (c-2): 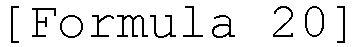 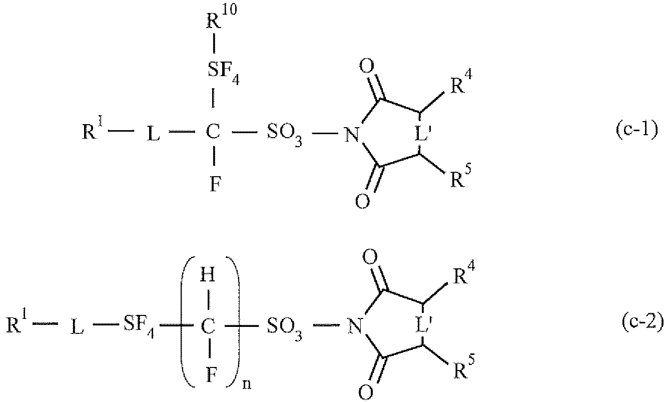 wherein R¹ represents an optionally substituted hydrocarbon group or heterocyclic group; L and L' are the same as or different from each other and each represents a single bond or a linking group. R⁴ and R⁵ are the same as or different from each other and each represent an optionally substituted hydrocarbon group; R⁴ and R⁵ are optionally linked to each other to form a ring together with an adjacent carbon atom; and R¹⁰ represents a fluorine atom, an aromatic hydrocarbon group, or an aromatic heterocyclic group, and n represents 0 or 1.

The imide sulfonate rapidly decomposes upon photoirradiation to generate a sulfonic acid.

When the imide sulfonate is a compound represented by formula (c-1), the sulfonic acid represented by formula (A-1) is generated.

When the imide sulfonate is a compound represented by formula (c-2), the sulfonic acid represented by formula (A-2) is generated.

The sulfonic acid has a SF₄R¹⁰ group or a SF₄ group and thus exhibits high acid strength.

In formulae (c-1) and (c-2), R¹, L, R¹⁰ and n are the same as R¹, L, R¹⁰ and n in formulae (a-1) and (a-2).

Specifically, R¹ is preferably a monovalent aliphatic hydrocarbon group or a monovalent aromatic heterocyclic group in order to provide excellent photosensitivity and generate a sulfonic acid with high acid strength, thereby producing an effect of improving the resolution of the photoresist.

Specifically, R¹⁰ is preferably a fluorine atom or an aromatic heterocyclic group, particularly preferably a fluorine atom in order to provide excellent photosensitivity and generate a sulfonic acid with high acid strength, thereby producing an effect of improving the resolution of the photoresist.

Examples of the linking group in L' are the same as those of the linking groups in L.

Specifically, L' is preferably a single bond in order to provide excellent photosensitivity and generate a sulfonic acid with high acid strength, thereby producing an effect of improving the resolution of the photoresist.

Examples of the hydrocarbon group in R⁴ and R⁵ are the same as those of the monovalent hydrocarbon group in R¹. Examples of the substituent that the hydrocarbon group in R⁴ and R⁵ may have are the same as those of the substituent that the monovalent hydrocarbon group in R¹ may have.

Examples of R⁴ and R⁵ that are optionally linked to each other to form a ring together with an adjacent carbon atom include an alicyclic hydrocarbon, an aromatic hydrocarbon ring, and a heterocyclic ring.

The alicyclic hydrocarbon is preferably a C₃₋₂₀ alicyclic hydrocarbon group, and examples thereof include C₃₋₁₂ cycloalkane rings such as cyclohexane and bicyclohexane; C₃₋₁₂ cycloalkene rings such as cyclohexene; and bridged rings such as norbornane, norbornene, adamantane, tricyclo[5.2.1.0^{2,6}]decane, tricyclo[4.3.1.1^{2,5}] undecane, tetracyclo[4.4.0.1^{2,5}.1^{7,10}]dodecane, and perhydro-1,4-methano-5,8-methanonaphthalene.

Examples of the aromatic hydrocarbon ring include benzene, naphthalene, and anthracene.

Examples of the heterocyclic ring are the same as those of the heterocyclic ring forming the heterocyclic group.

Specifically, the compound represented by formula (c-1) or (c-2) is preferably a compound represented by formula (c-1') or (c-2') in order to provide excellent photosensitivity and generate a sulfonic acid with high acid strength, thereby producing an effect of improving the resolution of the photoresist. In the following formulae, R¹, L, L', R⁴, and R⁵ are as defined above.

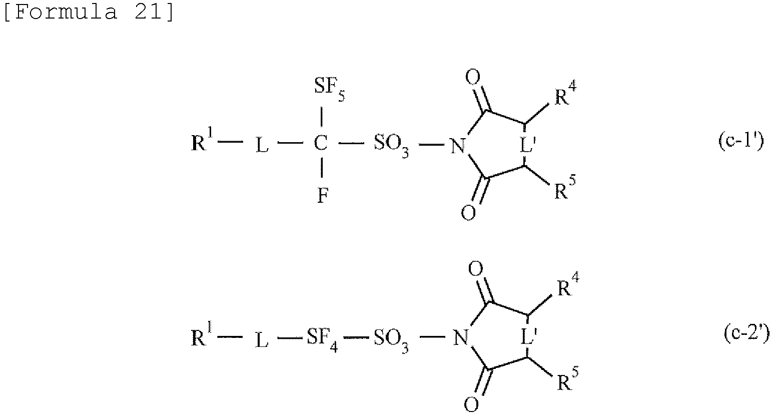

### Amide Sulfonate

An amide sulfonate of the present invention is a compound represented by formula (d-1) or (d-2): 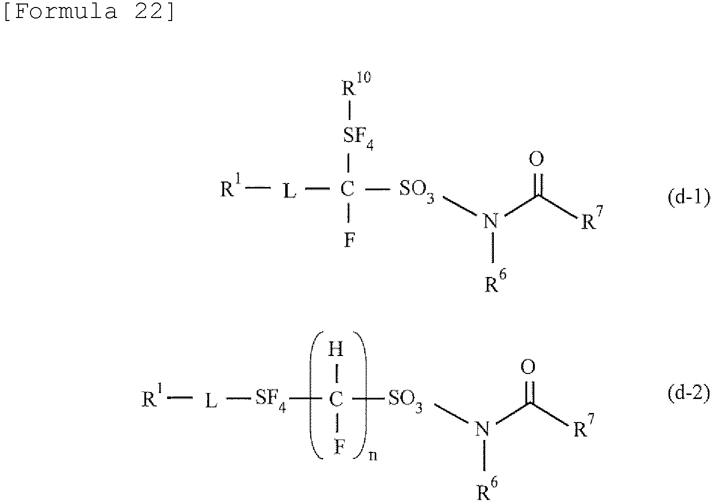 wherein R¹ represents an optionally substituted hydrocarbon group or heterocyclic group; L represents a single bond or a linking group; R⁶ and R⁷ are the same as or different from each other and each represent an optionally substituted hydrocarbon group; and R¹⁰ represents a fluorine atom, an aromatic hydrocarbon group, or an aromatic heterocyclic group, and n represents 0 or 1.

The amide sulfonate rapidly decomposes upon photoirradiation to generate a sulfonic acid.

When the amide sulfonate is a compound represented by formula (d-1), the sulfonic acid represented by formula (A-1) is generated.

When the imide sulfonate is a compound represented by formula (d-2), the sulfonic acid represented by formula (A-2) is generated.

The sulfonic acid has a SF₄R¹⁰ group or a SF₄ group and thus exhibits high acid strength.

In formulae (d-1) and (d-2), R¹, L, R¹⁰ and n are the same as R¹, L, R¹⁰ and n in formulae (a-1) and (a-2).

Specifically, R¹ is preferably a monovalent aliphatic hydrocarbon group or a monovalent aromatic heterocyclic group in order to provide excellent photosensitivity and generate a sulfonic acid with high acid strength, thereby producing an effect of improving the resolution of the photoresist.

Specifically, R¹⁰ is preferably a fluorine atom in order to provide excellent photosensitivity and generate a sulfonic acid with high acid strength, thereby producing an effect of improving the resolution of the photoresist.

Examples of the hydrocarbon group in R⁶ and R⁷ are the same as those of the monovalent hydrocarbon group in R¹. Examples of the substituent that the hydrocarbon group in R⁶ and R⁷ may have are the same as those of the substituent that the monovalent hydrocarbon group in R¹ may have.

Specifically, the compound represented by formula (d-1) or (d-2) is preferably a compound represented by formula (d-1') or (d-2') in order to provide excellent photosensitivity and generate a sulfonic acid with high acid strength, thereby producing an effect of improving the resolution of the photoresist. In the following formula, R¹, L, R⁶, and R⁷ are as defined above.

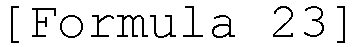

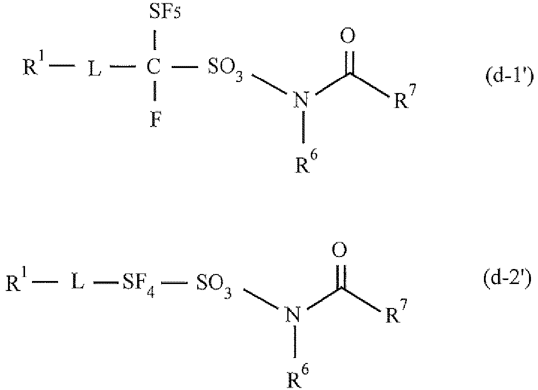

### Acid generator

An acid generator of the present invention contains the sulfonate, oxime sulfonate, imide sulfonate, or amide sulfonate, which rapidly decomposes upon photoirradiation to generate a sulfonic acid.

The acid generator has excellent solubility in a solvent (for example, PGMEA), and the amount of the acid generator (or the sulfonate, the oxime sulfonate, the imide sulfonate, or the amide sulfonate) to be dissolved in 100 parts by weight of PGMEA at normal temperature and pressure is, for example, 1 part by weight or more, preferably 5 parts by weight or more, and particularly preferably 10 parts by weight or more. Note that the upper limit is, for example, 30 parts by weight.

The acid generator also has excellent sensitivity to light and rapidly generates a sulfonic acid when irradiated with light. The wavelength of the light is, for example, 1 to 1000 nm. Examples of the light include infrared light, visible light, ultraviolet light, X-rays, electron beams, and extreme ultraviolet (EUV).

With the above-described characteristics, the acid generator can be suitably used as an acid generator for a photoresist (for example, a chemically amplified resist) or a cationic polymerization initiator for a cationically polymerizable compound.

### Photoresist

A photoresist of the present invention contains the acid generator and an acid-reactive compound.

The acid generator and the acid-reactive compound may be contained in the photoresist as separate compounds, or the acid-reactive compound may be incorporated into the acid generator and contained in the photoresist in an integrated state.

When the photoresist contains the acid generator and the acid-reactive compound as separate compounds, the content of the acid generator is, for example, 0.001 to 20% by weight, preferably 0.01 to 15% by weight, and particularly preferably 0.05 to 7% by weight of the content of the acid-reactive compound (the whole amount when two or more acid-reactive compounds are contained).

When the content of the acid generator is 0.001% by weight or more of the content of the acid-reactive compound, excellent sensitivity can be exhibited not only to light of a longer wavelength side but also to light having a wavelength of 20 nm or less. In addition, when the content is 20% by weight or less of the content of the acid-reactive compound, an effect of improving the resolution of the photoresist can be obtained.

In the case where the photoresist contains an integrated product of the acid generator and the acid-reactive compound, the content ratio of the acid generator and the acid-reactive compound in the photoresist is preferably in the same range as in the case where the acid generator and the acid-reactive compound are contained as separate compounds.

The acid-reactive compound is a compound having a property of changing solubility in an alkaline developing solution under the action of an acid and is a polymer. The photoresist of the present invention may contain one of the acid-reactive compounds alone or in combination with two or more thereof.

The acid-reactive compound includes a compound which is readily soluble in an alkaline developing solution and reacts with a crosslinking agent in the presence of acid to form a compound poorly soluble or insoluble in the alkaline developing solution and a compound which is poorly soluble or insoluble in the alkaline developing solution and exhibits increased solubility in an alkaline developing solution under the action of an acid.

Accordingly, the photoresist contains compositions (1) and (2) below. composition (1): A composition containing the acid generator and a negative photosensitive resin (QN) which is readily soluble in an alkaline developing solution and forms a compound poorly soluble or insoluble in the alkaline developing solution in the presence of acid Composition (2): A composition containing the acid generator and a positive photosensitive resin (QP) which is poorly soluble or insoluble in an alkaline developing solution and exhibits increased solubility in the alkaline developing solution under the action of an acid.

Examples of the negative photosensitive resin (or negative chemically amplified resin; QN) include a composition containing a phenolic hydroxy group-containing resin (QN1) and a crosslinking agent (QN2).

The phenolic hydroxy group-containing resin (QN1) refers to a resin that contains a phenolic hydroxy group, which is readily soluble in an alkaline developing solution and reacts with a crosslinking agent to become poorly soluble or insoluble in the alkaline developing solution, and examples thereof include a novolac resin, polyhydroxystyrene, a copolymer of hydroxystyrene, a copolymer of hydroxystyrene and styrene, a copolymer of hydroxystyrene, styrene, and a (meth)acrylic acid derivative, a phenol-xylylene glycol condensation resin, a cresol-xylylene glycol condensation resin, a phenolic hydroxy-containing polyimide, a phenolic hydroxy-containing polyamic acid, and a phenol-dicyclopentadiene condensation resin. The resin may be used singly or in combination with two or more thereof.

The phenolic hydroxyl group-containing resin (QN1) may contain a phenolic low-molecular-weight compound as a part of the components.

The weight-average-molecular weight (Mw) of the phenolic hydroxy group-containing resin (QN1) in terms of polystyrene measured by GPC is, for example, 2000 to 20000.

The crosslinking agent (QN2) is, for example, a compound capable of crosslinking the phenolic hydroxy group-containing resin (QN1) under the acids generated from the acid generators, and examples thereof include a bisphenol A-based epoxy compound, a bisphenol F-based epoxy compound, a bisphenol S-based epoxy compound, a novolac resin-based epoxy compound, a resol resin-based epoxy compound, poly(hydroxystyrene)-based epoxy compound, oxetane compound, a methylol group-containing melamine compound, a methylol group-containing benzoguanamine compound, a methylol group-containing urea compound, a methylol group-containing phenol compound, an alkoxyalkyl group-containing melamine compound, an alkoxyalkyl group-containing benzoguanamine compound, an alkoxyalkyl group-containing urea compound, an alkoxyalkyl group-containing phenol compound, a carboxymethyl group-containing melamine resin, a carboxymethyl group-containing benzoguanamine resin, a carboxymethyl group-containing urea resin, a carboxymethyl group-containing phenol resin, a carboxymethyl group-containing melamine compound, a carboxymethyl group-containing benzoguanamine compound, a carboxymethyl group-containing urea compound, and a carboxymethyl group-containing phenol compound. The compound may be used singly or in combination with two or more thereof.

The content of the crosslinking agent (QN2) is, for example, 10 to 40 mol% based on the total acidic functional groups in the phenolic hydroxy group-containing resin (QN1) from the viewpoint of efficiently making the phenolic hydroxy group-containing resin (QN1) poorly soluble or insoluble in an alkaline developing solution.

Examples of the positive photosensitive resin (or positive chemically amplified resin; QP) include an alkali-soluble resin into which an acid-dissociable group is introduced as a protective group (protective group-introduced resins; QP1).

The protective group-introduced resin (QP1) is a resin in which some or all of the hydrogen atoms of an acidic functional group (for example, a phenolic hydroxy group, a carboxyl group, and a sulfonyl group) in an alkaline soluble resin are substituted with an acid-dissociable group.

The protective group-introduced resin (QP1) itself is an insoluble or poorly soluble resin in an alkaline developing solution, and when the acid-dissociable group is dissociated by an acid (H⁺X⁻) generated from an acid generator, it changes into an alkali-soluble resin that is easily soluble in an alkaline developing solution.

The alkali-soluble resin is, for example, a resin having an HLB value of 4 to 19 (preferably 5 to 18, particularly preferably 6 to 17).

The alkali-soluble resin includes a phenolic hydroxy group-containing resin, a carboxyl group-containing resin, and a sulfonic acid group-containing resin.

Examples of the phenolic hydroxy group-containing resin include the same resin as the above phenolic hydroxy group-containing resin (QN1).

The carboxyl group-containing resin is not particularly limited as long as it is a polymer having a carboxyl group, and examples thereof include a homopolymer of a carboxyl group-containing vinyl monomer (Ba) and a copolymer of a carboxyl group-containing vinyl monomer (Ba) and a hydrophobic group-containing vinyl monomer (Bb).

The carboxyl group-containing vinyl monomer (Ba) is, for example, (meth)acrylic acid.

Examples of the hydrophobic group-containing vinyl monomer (Bb) include (meth)acrylic acid esters (Bb1) such as C₁₋₂₀ alkyl (meth)acrylate and an alicyclic group-containing (meth)acrylate, and aromatic hydrocarbon monomers (Bb2) such as a hydrocarbon monomer having a styrene skeleton and vinylnaphthalene.

The sulfonic acid group-containing resin is not particularly limited as long as it is a polymer having a sulfonic acid group, and is obtained, for example, by vinyl polymerization of a sulfonic acid group-containing vinyl monomer (Bc) such as vinyl sulfonic acid or styrene sulfonic acid and, if necessary, a hydrophobic group-containing vinyl monomer (Bb).

Examples of the acid-dissociable group contained in the protective group-introduced resin (QP1) include 1-substituted methyl groups such as a methoxymethyl group, a benzyl group, and a tert-butoxycarbonylmethyl group; 1-substituted ethyl groups such as a 1-methoxyethyl group and a 1-ethoxyethyl group; 1-branched alkyl groups such as a tert-butyl group; silyl groups such as a trimethylsilyl group; germyl groups such as a trimethylgermyl group; alkoxycarbonyl groups such as a tert-butoxycarbonyl group; acyl groups; and cyclic acid-dissociable groups such as a tetrahydropyranyl group, a tetrahydrofuranyl group, a tetrahydrothiopyranyl group, and a tetrahydrothiofuranyl group. One of these groups may be contained alone, or two or more thereof may be contained in combination.

The introduction ratio of the acid-dissociable group in the protective group-introduced polymer (QP1) (ratio of the number of acid-dissociable groups to the total number of unprotected acidic functional groups and acid-dissociable groups in the protective group-introduced polymer (QP1)) cannot be generally defined depending on the type of the acid-dissociable group or the alkali-soluble resin into which the groups are introduced, but is preferably 10 to 100%, and more preferably 15 to 100%.

The weight-average molecular weight (Mw) of the protective group-introduced polymer (QP1) in terms of polystyrene measured by GPC is, for example, 1000 to 150000, and preferably 3000 to 100000.

The photoresist of the present invention can be prepared, for example, by dissolving the acid generator in an organic solvent to mix the solution with a photosensitive resin.

If necessary, the photoresist of the present invention may contain one or two or more components other than the acid generator and the photosensitive resin. Examples of the other components include an organic solvent, a pigment, a dye, a photosensitizer, a dispersant, a surfactant, a filler, a leveling agent, an antifoaming agent, an antistatic agent, an ultraviolet absorber, a pH adjuster, a surface modifier, a plasticizer, and a drying accelerator.

The organic solvent may be any solvent that can dissolve the photosensitive resin and impart good coating properties to the photoresist, and specifically, a solvent having a boiling point of 200°C or lower is preferably used because the photoresist can be easily dried after being coated. Preferred examples of the organic solvent include aromatic hydrocarbons such as toluene; alcohols such as ethanol and methanol; ketones such as cyclohexanone, methyl ethyl ketone, and acetone; esters such as ethyl acetate, butyl acetate, and ethyl lactate; and glycol monoether monoesters such as propylene glycol monomethyl ether acetate (PGMEA). The compound may be used singly or in combination with two or more thereof.

Specifically, the organic solvent preferably contains at least one selected from the group consisting of ketone, ester (particularly, chain ester), and glycol monoether monoester.

The photoresist of the present invention contains a sulfonium salt having high sensitivity to light of wavelengths, including g-line (436 nm), h-line (405 nm), i-line (365 nm); excimer lasers such as KrF, ArF and F₂; and ultraviolet light, X-rays, electronic beams, and EUV. Therefore, when the photoresist of the present invention is used, a resist film having a fine pattern with high resolution can be produced by photolithography using the light of the wavelengths.

Examples of the method of forming a pattern by photolithography using the photoresist include a method including steps 1 to 3 below.
Step 1: forming a coating film of the photoresist on a substrate
Step 2: photo-irradiating the coating film to transfer a pattern
Step 3: performing alkaline development

### (Step 1)

This step involves forming a coating film of the photoresist on a substrate to be etched. The coating film of the photoresist can be formed by applying the photoresist to a substrate using a known method such as spin coating, curtain coating, roll coating, spray coating, or screen printing, followed by drying.

Although natural drying may be performed in a method of drying the photoresist, the photoresist can also be dried by heating (for example, heating at a temperature of not less than 50°C and less than 130°C for 1 to 5 minutes), which is excellent in workability, since the sulfonium salt has thermal stability.

The thickness of the coating film is, for example, 1 to 1000 nm.

### (Step 2)

This step is of photo-irradiating the coating film obtained through step 1 to transfer a pattern using a method such as photoirradiation through a photomask having the pattern.

The light to be used for photoirradiation is not particularly limited as long as it can generate an acid (H⁺X⁻, where X⁻ represents a counter anion) by decomposing the sulfonium salt contained in the coating film, but a short-wavelength light is preferably used from the viewpoint of making the pattern finer, and the wavelength of the light is, for example, preferably 450 nm or less (for example, 1 to 450 nm), more preferably 400 nm or less, still more preferably 300 nm or less, particularly preferably 200 nm or less, and most preferably 30 nm or less. Examples of the light include g-line (436 nm), h-line (405 nm), i-line (365 nm); excimer lasers such as KrF, ArF and F₂; and ultraviolet light, X-rays, electronic beams, and EUV.

After the photoirradiation, heating is preferably performed at a temperature of 60°C to 200°C for about 0.1 to 120 minutes from the viewpoint of increasing the difference in solubility in the alkaline developing solution between the exposed portion and the unexposed portion.

### (Step 3)

This step is of subjecting the coating film of the photoresist through step 2 to an alkali developing treatment.

Examples of the alkali developing solution to be used in the alkali developing treatment include an aqueous solution of sodium hydroxide, an aqueous solution of potassium hydroxide, sodium hydrogen carbonate, and an aqueous solution of tetramethylammonium salt.

Methanol, ethanol, isopropyl alcohol, tetrahydrofuran, N-methylpyrrolidone, or the like may be added to the alkaline developing solution.

The alkali developing treatment is performed by applying the alkaline developing solution to the coating film using a method such as a dip method, a shower method, or a spray method.

The temperature of the alkaline developing solution is, for example, 25 to 40°C. The alkali developing time is appropriately determined depending on the thickness of the coating film of the photoresist, and is, for example, about 1 to 5 minutes.

In the alkali developing treatment, it is preferred that the difference in solubility between the exposed portion and the unexposed portion of the coating film of the photoresist is large from the viewpoint of forming a fine pattern with good accuracy because the presence of a large amount of development residue is likely to cause a problem such as an abnormality in the shape of the interconnect. Since the photoresist of the present invention contains a sulfonium salt having a carboxyl group as described above, the developability of the resist is improved during alkali development, and the amount of development residue can be reduced. Therefore, defect-free products can be manufactured with high yield.

Through the step 3, a resist film having a fine pattern with good accuracy can be formed on the substrate. By etching the substrate using the resist film thus obtained, an electronic device or an optical device with high accuracy can be manufactured.

Examples of the electronic device include display devices such as an organic EL display and a liquid crystal display; input devices such as a touch screen; light-emitting devices; sensor devices; and micro electro-mechanical system (MEMS) devices such as an optical scanner, an optical switch, an acceleration sensor, a pressure sensor, a gyroscope, a micro flow path, and an inkjet head.

Examples of the optical device include an optical waveguide, a metalens, and a semiconductor laser.

### Cationic Polymerization Initiator

A cationic polymerization initiator of the present invention contains the sulfonate, oxime sulfonate, imide sulfonate, or amide sulfonate, which rapidly decomposes upon photoirradiation to generate a sulfonic acid.

The cationic polymerization initiator has excellent solubility in a solvent (for example, PGMEA), and the amount of the cationic polymerization initiator (or the sulfonate, the oxime sulfonate, the imide sulfonate, or the amide sulfonate) to be dissolved in 100 parts by weight of PGMEA at normal temperature and pressure is, for example, 1 part by weight or more, preferably 5 parts by weight or more, and particularly preferably 10 parts by weight or more. Note that the upper limit is, for example, 30 parts by weight.

The cationic polymerization initiator also has excellent sensitivity to light and rapidly generates a sulfonic acid when irradiated with the light. Note that the wavelength of the light is, for example, 1 to 1000 nm. Examples of the light include g-line (436 nm), h-line (405 nm), i-line (365 nm); excimer lasers such as KrF, ArF and F₂; and ultraviolet light, infrared light, visible light, ultraviolet light, X-rays, electron beams, and EUV.

### Curable Composition

A curable composition of the present invention includes the cationic polymerization initiator and a cationically polymerizable compound as a resin component, and each of the cationic polymerization initiator and the cationically polymerizable compound may be contained singly or in combination with two or more thereof.

The content of the cationic polymerization initiator is, for example, 0.05 to 20 parts by weight, preferably 0.1 to 10 parts by weight based on 100 parts by weight of the cationically polymerizable compound.

The cationically polymerizable compound refers to a compound having one or two or more cationically polymerizable groups selected from an epoxy group, an oxetanyl group, a vinyl ether group, and the like. Note that the epoxy group refers to a group containing a 3-membered cyclic ether skeleton, and the oxetanyl group refers to a group containing a 4-membered cyclic ether skeleton.

Examples of the cationically polymerizable compound include a compound having an epoxy group as a cationically polymerizable group (hereinafter referred to as an epoxy compound), a compound having an oxetanyl group as a cationically polymerizable group (hereinafter referred to as an oxetane compound), a compound having a vinyl ether group as a cationically polymerizable group (hereinafter referred to as a vinyl ether compound), a compound having an epoxy group and an oxetanyl group as cationically polymerizable groups, a compound having an epoxy group and a vinyl ether group as cationically polymerizable groups, and a compound having an oxetanyl group and a vinyl ether group as cationically polymerizable groups.

### Epoxy Compound

Examples of the epoxy compound include an epoxy-modified siloxane compound, an alicyclic epoxy compound (alicyclic epoxy resin), an aromatic epoxy compound (aromatic epoxy resin), and an aliphatic epoxy compound (aliphatic epoxy resin).

### Epoxy-modified Siloxane Compound

Examples of the epoxy-modified siloxane compound include epoxy-modified silicone and epoxy-modified polyorganosilsesquioxane.

### Alicyclic Epoxy Compound

Examples of the alicyclic epoxy compound include known or common compounds having one or more alicyclic rings and one or more epoxy groups in the molecule, examples of which include, but are not particularly limited to, the following compounds:
(1) a compound having an alicyclic epoxy group (i.e., an epoxy group composed of two adjacent carbon atoms and an oxygen atom forming an alicyclic ring in the molecule); and
(2) a compound having an alicyclic ring and a glycidyl ether group.

Examples of the compound (1) having an alicyclic epoxy group include 3,4-epoxycyclohexylmethyl-3,4-epoxycyclohexanecarboxylate, 3,4-epoxy-1-methylcyclohexyl-3,4-epoxy-1-methylhexanecarboxylate, 6-methyl-3,4-epoxycyclohexylmethyl-6-methyl-3,4-epoxycyclohexanecarboxylate, 3,4-epoxy-3-methylcyclohexylmethyl-3,4-epoxy-3-methylcyclohexanecarboxylate, 3,4-epoxy-5-methylcyclohexylmethyl-3,4-epoxy-5-methylcyclohexanecarboxylate, 2-(3,4-epoxycyclohexyl-5,5-spiro-3,4-epoxy)cyclohexanemetadioxane, bis(3,4-epoxycyclohexylmethyl)adipate, 3,4-epoxy-6-methylcyclohexylcarboxylate, methylenebis(3,4-epoxycyclohexane), dicyclopentadiene diepoxide, and ethylenebis(3,4-epoxycyclohexanecarboxylate) .

Examples of the compound (2) having an alicyclic ring and a glycidyl ether group include glycidyl ethers of an alicyclic alcohol(particularly, an alicyclic polyhydric alcohol). More specifically, examples thereof include compounds obtained by hydrogenating bisphenol A-type epoxy compounds (hydrogenated bisphenol A-type epoxy compounds), such as 2,2-bis[4-(2,3-epoxypropoxy)cyclohexyl]propane and 2,2-bis[3,5-dimethyl-4-(2,3-epoxypropoxy)cyclohexyl]propane; compounds obtained by hydrogenating bisphenol F-type epoxy compounds (hydrogenated bisphenol F-type epoxy compounds), such as bis[o,o-(2,3-epoxypropoxy)cyclohexyl]methane, bis[o,p-(2,3-epoxypropoxy)cyclohexyl]methane, bis[p,p-(2,3-epoxypropoxy)cyclohexyl]methane, and bis[3,5-dimethyl-4-(2,3-epoxypropoxy)cyclohexyl]methane; hydrogenated biphenol-type epoxy compounds; hydrogenated phenol novolac-type epoxy compounds; hydrogenated cresol novolac-type epoxy compounds; hydrogenated cresol novolac-type epoxy compounds of bisphenol A; hydrogenated naphthalene-type epoxy compounds; and hydrogenation products of trisphenolmethane-type epoxy compounds.

### Aromatic Epoxy Compound

Examples of the aromatic epoxy compound include an epibis-type glycidyl ether-type epoxy resin obtained by a condensation reaction of bisphenol (for example, bisphenol A, bisphenol F, bisphenol S, or fluorene bisphenol) and an epihalohydrin; a high-molecular-weight epibis-type glycidyl ether-type epoxy resin obtained by further addition reaction of these epibis-type glycidyl ether-type epoxy resins with the bisphenol; a novolac alkyl-type glycidyl ether-type epoxy resin obtained by further condensation reaction of a polyhydric alcohol obtained by a condensation reaction of a phenol (for example, phenol, cresol, xylenol, resorcin, catechol, bisphenol A, bisphenol F, or bisphenol S) and an aldehyde (for example, formaldehyde, acetaldehyde, benzaldehyde, hydroxybenzaldehyde, or salicylaldehyde) with an epihalohydrin; and an epoxy compound in which two phenol skeletons are bonded to the 9-position of a fluorene ring and glycidyl groups are each bonded to an oxygen atom obtained by removing a hydrogen atom from a hydroxy group of these phenol skeletons directly or via an alkyleneoxy group.

### Aliphatic Epoxy Compound

Examples of the aliphatic epoxy compound include a glycidyl ether of a q-hydric alcohol having no cyclic structure (where q is a natural number); a glycidyl ester of a monovalent or polyvalent carboxylic acid (for example, acetic acid, propionic acid, butyric acid, stearic acid, adipic acid, sebacic acid, maleic acid, or itaconic acid); an epoxidized product of fat and oil having a double bond, such as epoxidized linseed oil, epoxidized soybean oil, and epoxidized castor oil; and an epoxidized product of a polyolefin (including a polyalkadiene) such as epoxidized polybutadiene. Examples of the q-hydric alcohol having no cyclic structure include monohydric alcohols such as methanol, ethanol, 1-propyl alcohol, isopropyl alcohol, and 1-butanol; dihydric alcohols such as ethylene glycol, 1,2-propanediol, 1,3-propanediol, 1,4-butanediol, neopentyl glycol, 1,6-hexanediol, diethylene glycol, triethylene glycol, tetraethylene glycol, dipropylene glycol, polyethylene glycol, and polypropylene glycol; and trihydric or higher polyhydric alcohols such as glycerin, diglycerin, erythritol, trimethylolethane, trimethylolpropane, pentaerythritol, dipentaerythritol, and sorbitol. The q-hydric alcohol may be polyether polyol, polyester polyol, polycarbonate polyol, polyolefin polyol, or the like.

### Oxetane Compound

Examples of the oxetane compound include 3,3-bis(vinyloxymethyl)oxetane, 3-ethyl-3-hydroxymethyloxetane, 3-ethyl-3-(2-ethylhexyloxymethyl)oxetane, 3-ethyl-3-(hydroxymethyl)oxetane, 3-ethyl-3-[(phenoxy)methyl]oxetane, 3-ethyl-3-(hexyloxymethyl)oxetane, 3-ethyl-3-(chloromethyl)oxetane, 3,3-bis(chloromethyl)oxetane, 1,4-bis[(3-ethyl-3-oxetanylmethoxy)methyl]benzene, bis([1-ethyl(3-oxetanyl)]methyl)ether, 4,4'-bis[(3-ethyl-3-oxetanyl)methoxymethyl]bicyclohexyl, 4,4'-bis[3-ethyl-(3-oxetanyl)methoxymethyl]biphenyl, 1,4-bis[(3-ethyl-3-oxetanyl)methoxymethyl]cyclohexane, 1,4-bis([(3-ethyl-3-oxetanyl)methoxy]methyl)benzene, 3-ethyl-3([(3-ethyloxetane-3-yl)methoxy]methyl)oxetane, and xylylene bisoxetane.

### Vinyl Ether Compound

Examples of the vinyl ether compound include aryl vinyl ethers such as phenyl vinyl ether; alkyl vinyl ethers such as n-butyl vinyl ether and n-octyl vinyl ether; cycloalkyl vinyl ethers such as cyclohexyl vinyl ether; vinyl ethers having a hydroxy group such as 2-hydroxyethyl vinyl ether, diethylene glycol monovinyl ether, and 2-hydroxybutyl vinyl ether; and polyfunctional vinyl ethers such as hydroquinone divinyl ether, 1,4-butanediol divinyl ether, cyclohexane divinyl ether, cyclohexanedimethanol divinyl ether, ethylene glycol divinyl ether, diethylene glycol divinyl ether, and triethylene glycol divinyl ether.

The curable composition may contain one or two or more components other than the components above, if necessary. Examples of the other components include a sensitizer, a sensitizing aid, an antioxidant, a stabilizer, a surfactant, a solvent, a rheology control agent, a leveling agent, a silane coupling agent, a filler, a conductive particle, a polymerization inhibitor, a light stabilizer, a plasticizer, an antifoaming agent, a foaming agent, an ultraviolet absorber, a tackifier, a curing retarder, an ion adsorbent, a pigment, a dye, a phosphor, a release agent, an antistatic agent, a flame retardant, a radical polymerizable compound, a polyimide resin, a polyamide resin, a phenoxy resin, a poly(meth)acrylate resin, a polyurethane resin, a polyurea resin, a polyester resin, a polyvinyl butyral resin, SBS, and SEBS. The content thereof (the whole amount when two or more are contained) is, for example, about 0.05 to 50% by weight, preferably 0.05 to 10% by weight, and particularly preferably 0.1 to 5% by weight of the total amount (100% by weight) of the curable composition.

The curable composition can be produced by uniformly mixing the cationic polymerization initiator, the cationically polymerizable compound, and other components added, if necessary, using a generally known mixing device such as a rotation-revolution type stirring and defoaming device, a homogenizer, a planetary mixer, a three-roll mill, or a bead mill. Note that the components may be mixed simultaneously or sequentially.

The application of the curable composition is not particularly limited, and examples thereof include a paint, a coating agent, an ink, a positive-type resist, a resist film, a liquid resist, a photo-sensitive material, an adhesive, a forming material, a molding material, a putty, a glass fiber impregnant, a sealer, a sealing material, a sealant, and an optical modeling material.

Since the curable composition contains a cationic polymerization initiator that generates a sulfonic acid having an excellent acid strength, a cured product can be rapidly formed upon light irradiation. In addition, since the curable composition contains a cationic polymerization initiator with excellent solvent solubility, the cationic polymerization initiator is prevented from precipitating even when the composition is held under a low-temperature environment (for example, under a temperature environment of 0°C or less, preferably -25°C or less) after preparation. Therefore, there is a sufficient time after preparation until use, and the composition has excellent handling properties.

### Cured Product

A cured product of the present invention is of the curable composition.

The cured product is obtained by curing the curable composition.

The curable composition can be cured under irradiation with light. The light can be used without any particular limitation as long as it has the energy to induce the decomposition of the salt contained in the acid generator, but is preferably a light having a wavelength in the ultraviolet region to the visible light region obtained from a low-pressure, medium-pressure, high-pressure, or ultrahigh pressure mercury lamp, metal halide lamp, xenon lamp, carbon arc lamp, fluorescent lamp, solid-state semiconductor lasers, argon lasers, He-Cd lasers, KrF excimer lasers, ArF excimer lasers, F2 lasers, or the like. In addition, a radioactive ray having high energy such as an electron beam or an X-ray can also be used. The irradiation time of the light depends on the intensity of the energy beam and the permeability of the light with respect to the curable composition, but is usually about 0.1 to 10 seconds at room temperature. If necessary, after the light irradiation, a heat treatment may be performed at a temperature of room temperature to 150°C for several seconds to several hours.

As described above, each configuration of the present invention, a combination thereof, and the like are examples, and addition, omission, replacement, and modification of the configuration can be appropriately made without departing from the gist of the present invention.

### Examples

Hereinafter, the present invention will be described in more detail with reference to Examples, but the present invention is not limited to these Examples.

### Preparation Example 1 (Synthesis of Acid-Reactive Compound)

Under a nitrogen atmosphere, a flask was charged with 168.6 g of 2-ethyladamantan-2-yl methacrylate, 85.5 g of 3-hydroxy-1-adamantyl methacrylate, 172.1 g of 2-oxotetrahydrofuran-3-yl methacrylate, and 510 g of propylene glycol methyl ether acetate to obtain a monomer solution.

Then, 14.86 g of 2,2'-azobisisobutyronitrile, 2.6 g of 2-mercaptoethanol, and 127 g of propylene glycol methyl ether acetate were mixed together as an initiator solution.

Under a nitrogen atmosphere, another flask was charged with 292 g of propylene glycol methyl ether acetate and heated to 80°C with stirring, and then the monomer solution and the initiator solution were simultaneously added dropwise over 4 hours.

After completion of the dropwise addition, the reaction solution was stirred for 2 hours while maintaining the temperature at 80°C and then cooled to room temperature. The resulting polymerization solution was added dropwise to 12 kg of vigorously stirred methanol to separate the precipitated copolymer by filtration. The copolymer was washed twice with 3 kg of methanol and then vacuum-dried at 50°C for 20 hours to give 384 g of a white powder copolymer. When the obtained copolymer was analyzed by GPC, the weight average molecular weight (Mw) in terms of polystyrene was 6000.

The obtained copolymer was used as an acid-reactive compound.

### Example 1 (Production of Sulfonate)

To a reaction vessel were added 2.9 g of 3,3,4-trifluoro-4-(pentafluorosulfanyl)-1,2-oxathiethane-2,2-dioxide and 20 mL of hexane, followed by stirring. To the mixture was added 1.7 g of 1-adamantanemethanol in small portions. The reaction was then allowed to proceed for 5 hours at room temperature, and 10 mL of water was added thereto. An organic layer and an aqueous layer were separated by separatory operation. The resulting aqueous layer was extracted twice with 10 mL of diisopropyl ether, and the resulting organic layers were combined and washed three times with 10 mL of water. The solvent was evaporated under reduced pressure from the organic layer to give 3.8 g of white solid (yield: 87%). Based on ¹H-NMR and ¹⁹F-NMR, it was confirmed that the white solid obtained was a compound represented by formula (F-1) (hereinafter referred to as a compound (F-1)).

To a reaction vessel were added 4.3 g of the compound (F-1) and 50 mL of an acetonitrile-water mixed solvent (acetonitrile: water = 13:1 (weight ratio)) and dissolved. The mixture was cooled to 15°C in a water bath, to which 1.4 g of 15% aqueous sodium hydroxide solution was added in small portions. After the addition, the mixture was further stirred at room temperature for 1 hour. After the reaction solution was filtered, the filtrate was concentrated to give 2.3 g of white solid. To the resulting white solid were added 30 mL of diisopropyl ether and 25 mL of water, followed by stirring for 1 hour. To the aqueous layer separated after standing was added 30 mL of diisopropyl ether, followed by stirring for 1 hour. The aqueous layer separated after standing was extracted three times with 15 mL of propionitrile. The resulting organic solvent was combined, and the solvent was evaporated under reduced pressure to give 1.0 g of white solid (yield: 22%). Based on ¹H-NMR and ¹⁹F-NMR, it was confirmed that the resulting white solid was a compound represented by formula (F-2) (hereinafter referred to as a compound (F-2)).

To a reaction vessel were added 6.0 g of triphenylsulfonium chloride and 9.1 g of the compound (F-2), followed by 50 mL of dichloromethane and 50 mL of water, and the mixture was stirred at room temperature for 10 hours and allowed to stand still. Thereafter, the aqueous layer was removed by liquid separation, and the organic layer was washed five times with 50 mL of water. The organic layer was concentrated under reduced pressure, and the resulting solid was recrystallized with a dichloromethane-ether mixed solvent to give 12.5 g of white solid (yield: 90%). Based on ¹H-NMR and ¹⁹F-NMR, it was confirmed that the white solid obtained was triphenylsulfonium 1-adamantanemethyloxycarbonyl-1-fluoro-1-(pentafluorosulfanyl)-methanesulfonate represented by formula (S-1a) below.

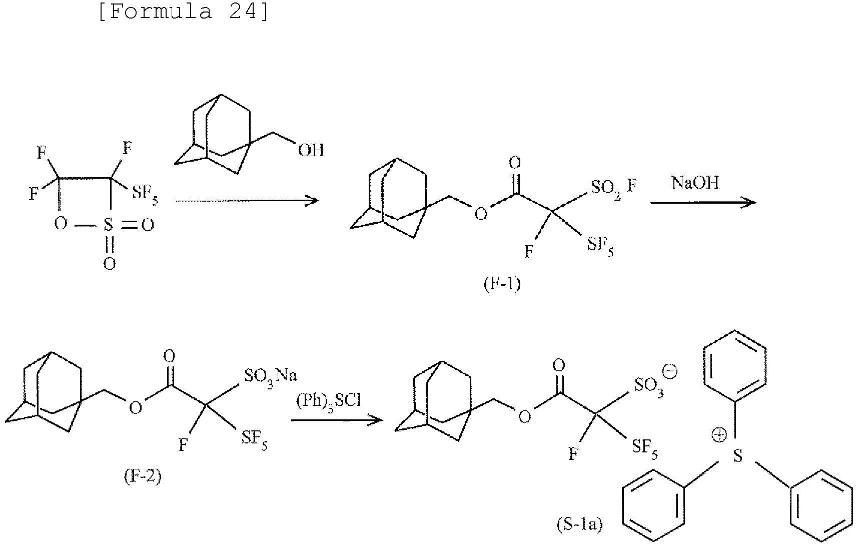

### Example 2

The same procedure as in Example 1 was performed except that 6.0 g of triphenylsulfonium chloride was changed to 8.6 g of bis(4-tert-butylphenyl)iodonium chloride to give 14.2 g of white solid (yield: 86%). Based on ¹H-NMR and ¹⁹F-NMR, it was confirmed that the white solid obtained was bis(4-tert-butylphenyl)iodonium 1-adamantanemethyloxycarbonyl-1-fluoro-1-(pentafluorosulfanyl)-methanesulfonate.

### Example 3

To a reaction vessel were added 2.9 g of 3,3,4-trifluoro-4-(pentafluorosulfanyl)-1,2-oxathiethane-2,2-dioxide and 20 mL of hexane, followed by stirring. To the mixture was added 0.5 g of ethanol in small portions. The reaction was then allowed to proceed for 5 hours at room temperature, and 10 mL of water was added thereto. An organic layer and an aqueous layer were separated by separatory operation. The resulting aqueous layer was extracted twice with 10 mL of diisopropyl ether, and the resulting organic layers were combined and washed three times with 10 mL of water. The solvent was evaporated under reduced pressure from the organic layer to give 2.4 g of white solid (yield: 77%). Based on ¹H-NMR and ¹⁹F-NMR, it was confirmed that the white solid obtained was 1-ethoxycarbonyl-1-fluoro-1-(pentafluorosulfanyl)-methanesulfonyl fluoride (hereinafter referred to as a compound (F-3)).

To a reaction vessel was added 3.1 g of the compound (F-3), and the reaction vessel was purged with nitrogen, to which 30 mL of toluene was added, and the mixture was stirred. The reaction vessel was cooled in a dry ice-acetone bath to -78°C, to which 10 mL of a 1M toluene solution of diisobutylaluminum hydride was added dropwise with stirring. After the dropwise addition, the mixture was stirred for 1 hour. The bath was removed, the temperature was gradually returned to room temperature, and the reaction was continued for another 1 hour at room temperature. Thereafter, 7 mL of methanol was added dropwise, and the mixture was stirred for 30 minutes, to which 15 mL of a 30% aqueous sodium potassium tartrate solution was added, and the mixture was stirred for another 30 minutes. A toluene layer and an aqueous layer were separated by separatory operation. The toluene layer was washed twice with 5 mL of a 30% aqueous sodium potassium tartrate solution. The aqueous layer was extracted with 10 mL of toluene. The solvent was concentrated under reduced pressure from the organic layer to give 1.8 g of white solid (yield: 67%). Based on ¹H-NMR and ¹⁹F-NMR, it was confirmed that the white solid obtained was 2-hydroxy-1-fluoro-1-(pentafluorosulfanyl)-ethanesulfonyl fluoride (hereinafter referred to as a compound (F-4)).

A reaction vessel was charged with 2.7 g of the compound (F-4), and 5 mL of acetonitrile and 50 mL of water were added to dissolve the compound, to which 4.0 g of 10% aqueous sodium hydroxide solution was added. The mixture was stirred at room temperature for 5 hours, gradually added to 500 mL of acetone, and then further stirred for another hour. The precipitated solid was filtered to obtain 2.2 g of white solid (yield: 76%). Based on ¹H-NMR and ¹⁹F-NMR, it was confirmed that the white solid was sodium 2-hydroxy-1-fluoro-1-(pentafluorosulfanyl)-ethanesulfonate (hereinafter referred to as a compound (F-5)).

To a reaction vessel were added 2.9 g of the compound (F-5) and 2.0 g of 1-adamantane carboxylic acid chloride, and the reaction vessel was purged with nitrogen, to which 30 mL of acetonitrile was added. The mixture was stirred and then cooled to 0°C in an ice bath, and 1.1 g of 2,6-lutidine was added thereto dropwise. Then, the mixture was stirred at room temperature for 8 hours. After the reaction, 30 mL of water and 50 mL of dichloromethane were added and stirred for 1 hour. After removal of the aqueous layer by liquid separation, the organic layer was washed twice with 10 mL of water, and the solvent was evaporated under reduced pressure. The resulting solid was recrystallized with 2-propanol to give 2.4 g of white solid (yield: 53%). Based on ¹H-NMR and ¹⁹F-NMR, it was confirmed that the white solid obtained was sodium 2-(1-adamantanecarbonyloxy)-1-fluoro-1-(pentafluorosulfanyl)-ethanesulfonate (hereinafter referred to as a compound (F-6)).

To a reaction vessel were added 6.0 g of triphenylsulfonium chloride and 9.1 g of the compound (F-6), followed by 50 mL of dichloromethane and 50 mL of water, and the mixture was stirred at room temperature for 10 hours and allowed to stand still. Thereafter, the aqueous layer was removed by liquid separation, and the organic layer was washed five times with 50 mL of water. The organic layer was concentrated under reduced pressure, and the resulting solid was recrystallized with a dichloromethane-ether mixed solvent to give 12.2 g of white solid (yield: 88%). Based on ¹H-NMR and ¹⁹F-NMR, it was confirmed that the white solid obtained was triphenylsulfonium 2-(1-adamantanecarbonyloxy)-1-fluoro-1-(pentafluorosulfanyl)-ethanesulfonate.

### Example 4

To a reaction vessel were added 2.9 g of 3,3,4-trifluoro-4-(pentafluorosulfanyl)-1,2-oxathiethane-2,2-dioxide and 20 mL of hexane, followed by stirring. To the mixture was added 0.7 g of tert-butanol in small portions. The reaction was then allowed to proceed for 5 hours at room temperature, and 10 mL of water was added thereto. An organic layer and an aqueous layer were separated by separatory operation. The resulting aqueous layer was extracted twice with 10 mL of diisopropyl ether, and the resulting organic layers were combined and washed three times with 10 mL of water. The solvent was evaporated under reduced pressure from the organic layer to give 3.0 g of white solid (yield: 88%). Based on ¹H-NMR and ¹⁹F-NMR, it was confirmed that the white solid obtained was 1-tert-butoxycarbonyl-1-fluoro-1-(pentafluorosulfanyl)-methanesulfonyl fluoride (hereinafter referred to as a compound (F-7)).

To a reaction vessel were added 3.4 g of the compound (F-7) and 50 mL of a mixed solvent of acetonitrile/water (13:1 by weight) and dissolved. The mixture was cooled to 15°C in a water bath, to which 1.4 g of 15% aqueous sodium hydroxide solution was added in small portions. After the addition, the mixture was further stirred at room temperature for 1 hour. After the reaction solution was filtered, the filtrate was concentrated to give 2.3 g of white solid, to which 30 mL of diisopropyl ether and 25 mL of water was added. The mixture was stirred for 1 hour. To the aqueous layer separated after standing was added 30 mL of diisopropyl ether, followed by stirring for 1 hour. The aqueous layer separated after standing was extracted three times with 15 mL of propionitrile. The resulting organic solvent was combined, and the solvent was evaporated under reduced pressure to give 1.0 g of white solid (yield: 72%). Based on ¹H-NMR and ¹⁹F-NMR, it was confirmed that the white solid obtained was sodium 1-tert-butoxycarbonyl-1-fluoro-1-(pentafluorosulfanyl)-methanesulfonate (hereinafter referred to as a compound (F-8)).

To a reaction vessel was added 3.6 g of the compound (F-8), and the compound was dissolved in 50 mL of propionitrile, to which 3.0 g of p-toluenesulfonic acid monohydrate was added. The mixture was stirred under reflux for 4 hours and then cooled to room temperature. The solid was filtered, and the filtrate was concentrated under reduced pressure. The obtained solid was washed with diethyl ether and dried to give 2.3 g of white solid (yield: 75%). Based on ¹H-NMR and ¹⁹F-NMR, it was confirmed that the white solid obtained was sodium 1-hydroxycarbonyl-1-fluoro-1-(pentafluorosulfanyl)-methanesulfonate (hereinafter referred to as a compound (F-9)).

To a reaction vessel were added 3.1 g of the compound (F-9), 2.3 g of ethyldiisopropylaminocarbodiimide hydrochloride, and 0.1 g of dimethylaminopyridine, and the reaction vessel was purged with nitrogen, to which 50 mL of THF was added. The mixture was stirred and then cooled to 0°C in an ice bath, to which 1.3 g of 2-hydroxyethyl methacrylate was added. The mixture was then stirred at room temperature for 12 hours. Thereafter, 10 mL of water was added to stop the reaction, the solvent was evaporated under reduced pressure, and the mixture was extracted three times with 50 mL of ethyl acetate. The resulting organic layer was washed with saturated sodium carbonate and further washed five times with water, and the solvent was evaporated under reduced pressure from the organic layer to give 2.1 g of light yellow solid (yield: 50%). Based on ¹H-NMR and ¹⁹F-NMR, it was confirmed that the light yellow solid obtained was sodium 1-[2-(methacryloxy)ethyloxycarbonyl]-1-fluoro-1-(pentafluorosulfanyl)-methanesulfonate (hereinafter referred to as a compound (F-10)).

To a reaction vessel were added 6.0 g of triphenylsulfonium chloride and 9.1 of the compound (F-10), followed by 50 mL of dichloromethane and 50 mL of water, and the mixture was stirred at room temperature for 10 hours and allowed to stand still. Thereafter, the aqueous layer was removed by liquid separation, and the organic layer was washed five times with 50 mL of water. The organic layer was concentrated under reduced pressure, and the resulting solid was recrystallized with a dichloromethane-ether mixed solvent to give 8.7 g of light yellow solid (yield: 66%). Based on ¹H-NMR and ¹⁹F-NMR, it was confirmed that the light yellow solid obtained was triphenylsulfonium 1-[2-(methacryloxy)ethyloxycarbonyl]-1-fluoro-1-(pentafluorosulfanyl)methanesulfonate.

### Examples 5 and 6

A compound listed in the table was synthesized according to Example 1.

### Example 7

To a reaction vessel were added, 4.5 g of 1-adamantanemethyloxycarbonyl-1-fluoro-1-(pentafluorosulfanyl)-methanesulfonyl chloride and 1.7 g of N-hydroxyphthalimide, and the mixture was dissolved in 50 mL of chloroform and then cooled to 0°C. To the mixture was slowly added 1.1 g of triethylamine dropwise with stirring. Thereafter, the temperature was raised, and the mixture was stirred at 50°C for 8 hours, and 50 mL of chloroform and 50 mL of water were then added to the reaction solution and stirred for 1 hour. After allowing to stand still, the aqueous layer was removed, and the organic layer was removed under reduced pressure to give a yellow oil. Further, recrystallization was performed with methanol to give 3.1 g of light yellow solid (yield: 54%). Based on ¹H-NMR and ¹⁹F-NMR, it was confirmed that the light yellow solid obtained was N-[1-adamantanemethyloxycarbonyl-1-fluoro-1-(pentafluorosulfanyl)-methanesulfonyloxy]phthalimide.

### Example 8

The same procedure as in Example 7 was performed except that 1.7 g of N-hydroxyphthalimide was changed to 1.8 g of 4-methoxybenzoylcyanide oxime to give 3.4 g of light yellow solid (yield: 58%). Based on ¹H-NMR and ¹⁹F-NMR, it was confirmed that the light yellow solid obtained was N-[1-adamantanemethyloxycarbonyl-1-fluoro-1-(pentafluorosulfanyl)-methanesulfonyloxy]-4-methoxybenzimidoylcyanide.

### Example 9

To a reaction vessel was added 1.3 g of N-methylhydroxylammonium chloride, the compound was dissolved in 10 mL of methanol, and 15 mL of a 10% methanol solution of potassium hydroxide was added dropwise with stirring at 0°C. Furthermore, 2.4 g of 8-methoxycoumarin-3-carboxylic acid chloride dissolved in 10 mL of THF was added and stirred for 1 hour. The reaction solution was returned to room temperature, stirred for another 1 hour, and then evaporated under reduced pressure. The residue was extracted with 20 mL of ethyl acetate and 20 mL of saturated saline, the organic layer was separated, and the solvent was then evaporated to give 1.8 g of white solid (yield: 72%). Based on ¹H-NMR, it was confirmed that the white solid obtained was N-methyl-N-hydroxy-8-methoxycoumarin-3-carbonylamide (hereinafter referred to as a compound (F-11)).

The same procedure as in Example 7 was performed except that 1.7 g of N-hydroxyphthalimide was changed to 2.5 g of the compound (F-11) to give 3.6 g of light yellow solid (yield: 54%). Based on ¹H-NMR and ¹⁹F-NMR, it was confirmed that the light yellow solid obtained was N-methyl-N-[1-adamantanemethyloxycarbonyl-1-fluoro-1-(pentafluorosulfanyl)-methanesulfonyloxy]-8-methoxycoumarin-3-carbonylamide.

### Example 10

To a reaction vessel were added 3.0 g of 5-bromopyridin-2-yltetrafluoro-λ⁶-sulfanyl chloride, 200 mL of acetonitrile, 10 g of sodium dithionite, and 0.25 g of benzoyl peroxide to carry out the reaction at 85°C for 65 hours. The solvent was evaporated under reduced pressure, to which 20 mL of water and 20 mL of toluene were added, and the mixture was stirred and allowed to stand for 1 hour. The aqueous layer was separated, to which 100 mL of saturated saline was added, and the precipitated solid was filtered and recrystallized with a methanol-acetone mixed solvent to give 1.1 g of light yellow solid (yield: 30%). Based on ¹H-NMR and ¹⁹F-NMR, it was confirmed that the light yellow solid obtained was sodium (5-bromopyridin-2-yltetrafluoro-λ⁶-sulfanyl)sulfonate (hereinafter referred to as a compound (F-12)).

To a reaction vessel were added 6.0 g of triphenylsulfonium chloride and 7.4 g of the compound (F-12), followed by 50 mL of dichloromethane and 50 mL of water, and the mixture was stirred at room temperature for 10 hours and allowed to stand still. Thereafter, the aqueous layer was removed by liquid separation, and the organic layer was washed five times with 50 mL of water. The organic layer was concentrated under reduced pressure, and the resulting solid was recrystallized with a dichloromethane-ether mixed solvent to give 9.0 g of light yellow solid (yield: 74%). Based on ¹H-NMR and ¹⁹F-NMR, it was confirmed that the light yellow solid obtained was triphenylsulfonium (5-bromopyridin-2-yltetrafluoro-λ⁶-sulfanyl) sulfonate.

### Example 11

To a reaction vessel were added 2.6 g of 4-chlorophenyltetrafluoro-λ⁶-sulfanyl chloride, 200 mL of acetonitrile, 10 g of sodium dithionite, and 0.25 g of benzoyl peroxide to carry out the reaction at 85°C for 65 hours. The solvent was evaporated under reduced pressure, to which 20 mL of water and 20 mL of toluene were added, and the mixture was stirred and allowed to stand for 1 hour. The aqueous layer was separated, to which 100 mL of saturated saline was added, and the precipitated solid was filtered and recrystallized with a methanol-acetone mixed solvent to give 1.4 g of white solid (yield: 44%). Based on ¹H-NMR and ¹⁹F-NMR, it was confirmed that the white solid obtained was sodium (4-chlorophenyltetrafluoro-λ⁶-sulfanyl)sulfonate (hereinafter referred to as a compound (F-13)).

To a reaction vessel were added 6.0 g of triphenylsulfonium chloride and 6.4 g of the compound (F-13), followed by 50 mL of dichloromethane and 50 mL of water, and the mixture was stirred at room temperature for 10 hours and allowed to stand still. Thereafter, the aqueous layer was removed by liquid separation, and the organic layer was washed five times with 50 mL of water. The organic layer was concentrated under reduced pressure, and the resulting solid was recrystallized with a dichloromethane-ether mixed solvent to give 9.0 g of white solid (yield: 80%). Based on ¹H-NMR and ¹⁹F-NMR, it was confirmed that the white solid obtained was triphenylsulfonium (4-chlorophenyltetrafluoro-λ⁶-sulfanyl)sulfonate.

### Example 12

To a reaction vessel were added 2.3 g of 4-fluorophenyltetrafluoro-λ⁶-sulfanyl chloride, 200 mL of acetonitrile, 10 g of sodium dithionite, and 0.25 g of benzoyl peroxide to carry out the reaction at 85°C for 65 hours. The solvent was evaporated under reduced pressure, to which 20 mL of water and 20 mL of toluene were added, and the mixture was stirred for 1 hour and allowed to stand. The aqueous layer was separated, to which 100 mL of saturated saline was added, and the precipitated solid was filtered and recrystallized with a methanol-acetone mixed solvent to give 1.1 g of white solid (yield: 36%). Based on ¹H-NMR and ¹⁹F-NMR, it was confirmed that the white solid was sodium (4-fluorophenyltetrafluoro-λ⁶-sulfanyl)sulfonate (hereinafter referred to as a compound (F-14)).

To a reaction vessel were added 6.0 g of triphenylsulfonium chloride and 6.1 g of the compound (F-14), followed by 50 mL of dichloromethane and 50 mL of water, and the mixture was stirred at room temperature for 10 hours and allowed to stand still. Thereafter, the aqueous layer was removed by liquid separation, and the organic layer was washed five times with 50 mL of water. The organic layer was concentrated under reduced pressure, and the resulting solid was recrystallized with a dichloromethane-ether mixed solvent to give 7.3 g of white solid (yield: 67%). Based on ¹H-NMR and ¹⁹F-NMR, it was confirmed that the white solid obtained was triphenylsulfonium (4-fluorophenyltetrafluoro-λ⁶-sulfanyl)sulfonate.

### Examples 13 and 14

A compound listed in the table was synthesized according to Example 1.

### Example 15

A compound listed in the table was synthesized according to Example 7.

### Example 16

To a reaction vessel was added 3.6 g of 3,3,4-trifluoro-4-(p-fluorophenyltetrafluoro-λ⁶-sulfanyl)-1,2-oxathietane-2,2-dioxide and 15 mL of water to carry out the reaction at 60°C for 60 hours. The reaction vessel was thoroughly cooled in a salt-ice bath and dried under reduced pressure to give a pale yellow solid.

To the pale yellow solid obtained were added 40 mL of dry methanol and 4.5 g of lithium hydroxide monohydrate at room temperature, followed by stirring for 16 hours. The reaction was then performed under reflux for 6 hours.

The reaction vessel was thoroughly cooled in a salt-ice bath and dried under reduced pressure to give a pale yellow solid. The pale yellow solid obtained was dissolved in 50 mL of THF and filtered. The filtrate was concentrated, to which hexane was added to precipitate a solid, and the precipitated solid was filtered off to give 1.6 g of pale yellow solid (yield: 50%). Based on ¹H-NMR and ¹⁹F-NMR, it was confirmed that the pale yellow solid obtained was lithium 1-fluoro-1-(p-fluorophenyltetrafluoro-λ⁶-sulfanyl)-methanesulfonate (hereinafter referred to as a compound (F-15)).

To a reaction vessel were added 3.0 g of triphenylsulfonium chloride and 3.2 g of the compound (F-15), followed by 20 mL of dichloromethane and 20 mL of water, and the mixture was stirred at room temperature for 10 hours and allowed to stand still. Thereafter, the aqueous layer was removed by liquid separation, and the organic layer was washed five times with 20 mL of water. The organic layer was concentrated under reduced pressure, and the resulting solid was recrystallized with a dichloromethane-ether mixed solvent to give 5.0 g of pale yellow solid (yield: 87%). Based on ¹H-NMR and ¹⁹F-NMR, it was confirmed that the pale yellow solid obtained was triphenylsulfonium 1-fluoro-1-(p-fluorophenyltetrafluoro-λ⁶-sulfanyl)methanesulfonate.

### Examples 17 to 22

Compounds listed in the tables were synthesized by the method according to Example 16.

### Examples 23 to 24

Compounds listed in the tables were synthesized by the method according to Example 1.

### Examples 25, 28, 31, and 32

Compounds listed in the tables were synthesized by the method according to Example 7.

### Examples 26 and 29

Compounds listed in the tables were synthesized by the method according to Example 9.

### Examples 27 and 30

Compounds listed in the tables were synthesized by the method according to Example 8.

The compounds obtained in Examples or the compounds listed in the tables as Comparative Examples or Reference Examples were used as acid generators to prepare a photoresist according to the method described below, and the optimum exposure dose, resolution, and pattern shape were measured using the obtained photoresist.

### Preparation of Photoresist

After 7.4 parts by weight of the compounds in Examples, Comparative Examples, or Reference Examples, 1.1 parts by weight of tris[2-(methoxymethoxy)ethyl]amine, 896 parts by weight of PGMEA, and 364 parts by weight of cyclohexanone were mixed with 80 parts by weight of the acid-reactive compound obtained in Preparation Example 1 to obtain a mixture, the obtained mixture was filtered through a 0.2 µm Teflon ^{®} filter to prepare a photoresist.

### Exposure and Development

A silicon substrate was coated with an anti-reflective coating solution (ARC-29A, manufactured by Nissan Chemical Industries, Ltd.) and baked at 200°C for 60 seconds to prepare an anti-reflective coating (78 nm in thickness).

The photoresist obtained through the preparation was spin-coated on the substrate with the anti-reflective coating and baked at 120°C for 60 seconds using a hot plate to prepare a resist film having a thickness of 160 nm.

The obtained resist film was exposed using an ArF excimer laser microstepper (NSR-S307E, manufactured by Nikon Corporation, NA = 0.85, 4/5 annular illumination, Cr mask), baked at 100°C for 60 seconds, and then immersed in an aqueous solution of 2.38% by mass tetramethylammonium hydroxide for 60 seconds for development.

### Evaluation of Optimum Exposure Dose, Resolution, and Pattern Shape

The optimum exposure dose (Eop, mJ/cm²) was defined as the exposure dose required to resolve an 80 nm line- and-space pattern at 1:1, and the resolution was defined as the minimum line width (nm) of the separated line-and-space pattern at this exposure dose.

In addition, the shape of the pattern on the resist cross-section was observed using a scanning electron microscope.

### Evaluation of Gas Phase Acidity

The gas phase acidity ΔG (kcal/mol) of sulfonic acid generated from the compounds of Examples, Comparative Examples, or Reference Examples was calculated by DFT calculation (B3LYP/6-311 + G (d, p)) using quantum chemical calculation software (Gaussian 16 program).

### [Table 1]

**Table 1**

| | Acid generator | Gas phase acidity ΔG (kcal/mol) | Optimum exposure dose (mJ/cm²) | Resolution (nm) | Pattern shape |
|---|---|---|---|---|---|
| Example 1 | 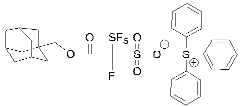 | 284.1 | 28 | 60 | Rectangle |
| Example 2 | 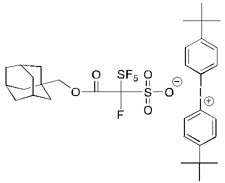 | 284.1 | 25 | 63 | Rectangle |
| Example 3 | 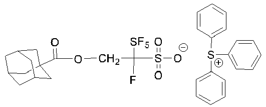 | 285.2 | 30 | 60 | Rectangle |
| Example 4 | 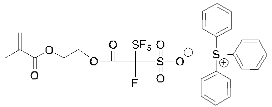 | 284.1 | 30 | 60 | Rectangle |

### [Table 2]

**Table 2**

| | Acid generator | Gas phase acidity ΔG (kcal/mol) | Optimum exposure dose (mJ/cm²) | Resolution (nm) | Pattern shape |
|---|---|---|---|---|---|
| Example 5 | 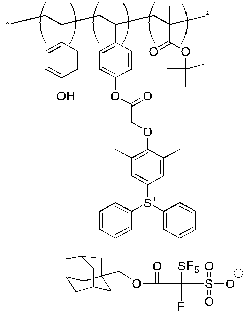 | 284.1 | 32 | 61 | Rectangle |
| Example 6 | 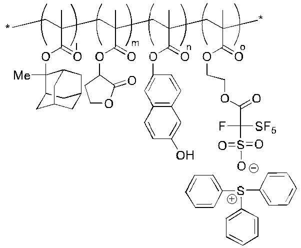 | 284.1 | 33 | 60 | Rectangle |

### [Table 3]

**Table 3**

| | Acid generator | Gas phase acidity ΔG (kcal/mol) | Optimum exposure dose (mJ/cm²) | Resolution (nm) | Pattern shape |
|---|---|---|---|---|---|
| Example 7 | 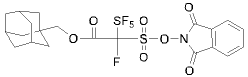 | 284.1 | 33 | 63 | Rectangle |
| Example 8 | 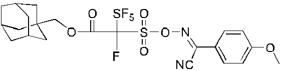 | 284.1 | 32 | 63 | Rectangle |
| Example 9 | 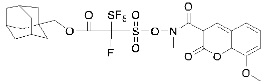 | 284.1 | 33 | 62 | Rectangle |
| Example 10 | 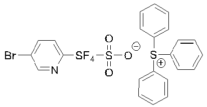 | 272.3 | 31 | 63 | Rectangle |
| Example 11 | 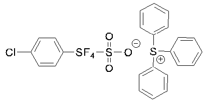 | 270 | 30 | 64 | Rectangle |
| Example 12 | 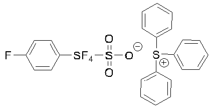 | 270.8 | 31 | 63 | Rectangle |

### [Table 4]

**Table 4**

| | Acid generator | Gas phase acidity ΔG (kcal/mol) | Optimum exposure dose (mJ/cm²) | Resolution (nm) | Pattern shape |
|---|---|---|---|---|---|
| Reference Example 1 | 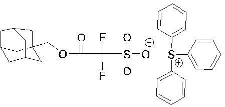 | 297.3 | 33 | 70 | Rectangle |
| Reference Example 2 | 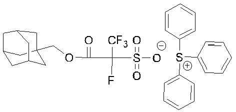 | 291.5 | 33 | 70 | Rectangle |
| Comparative Example 1 | 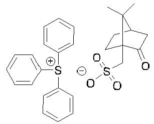 | 312.6 | No pattern obtained | - | - |

### [Table 5]

**Table 5**

| | Acid generator | Gas phase acidity ΔG (kcal/mol) | Optimum exposure dose (mJ/cm²) | Resolution (nm) | Pattern shape |
|---|---|---|---|---|---|
| Example 13 | 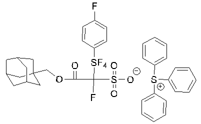 | 291.2 | 32 | 68 | Rectangle |
| Example 14 | 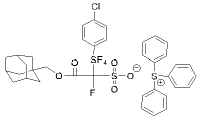 | 290.2 | 32 | 67 | Rectangle |
| Example 15 | 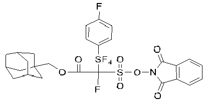 | 291.2 | 33 | 67 | Rectangle |
| Example 16 | 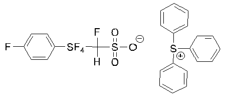 | 293.7 | 32 | 68 | Rectangle |
| Example 17 | 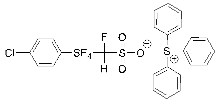 | 293.4 | 32 | 68 | Rectangle |

### [Table 6]

**Table 6**

| | Acid generator | Gas phase acidity ΔG (kcal/mol) | Optimum exposure dose (mJ/cm²) | Resolution (nm) | Pattern shape |
|---|---|---|---|---|---|
| Example 18 | 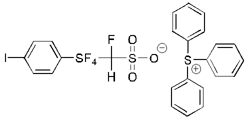 | 293.3 | 32 | 68 | Rectangle |
| Example 19 | 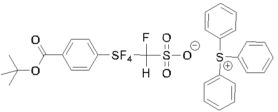 | 293.8 | 32 | 65 | Rectangle |
| Example 20 | 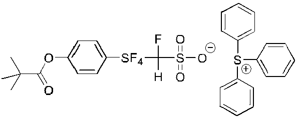 | 295.0 | 32 | 67 | Rectangle |
| Example 21 | 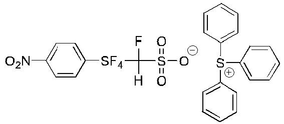 | 288.9 | 31 | 64 | Rectangle |
| Example 22 | 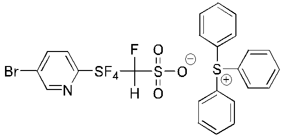 | 295.1 | 32 | 67 | Rectangle |

### [Table 7]

**Table 7**

| | Acid generator | Gas phase acidity ΔG (kcal/mol) | Optimum exposure dose (mJ/cm²) | Resolution (nm) | Pattern shape |
|---|---|---|---|---|---|
| Example 23 | 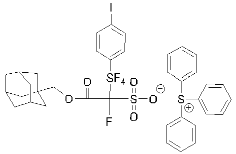 | 290.6 | 32 | 67 | Rectangle |
| Example 24 | 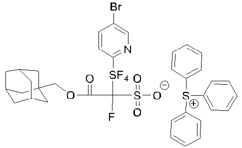 | 292.5 | 31 | 66 | Rectangle |
| Example 25 | 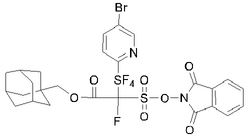 | 292.5 | 33 | 68 | Rectangle |
| Example 26 | 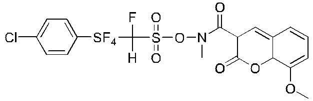 | 293.4 | 33 | 67 | Rectangle |
| Example 27 | 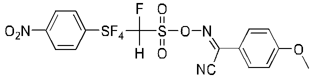 | 288.9 | 32 | 66 | Rectangle |

### [Table 8]

**Table 8**

| | Acid generator | Gas phase acidity ΔG (kcal/mol) | Optimum exposure dose (mJ/cm²) | Resolution (nm) | Pattern shape |
|---|---|---|---|---|---|
| Example 28 | 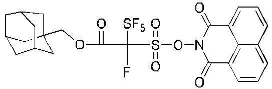 | 284.1 | 31 | 63 | Rectangle |
| Example 29 | 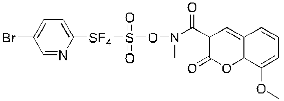 | 272.3 | 32 | 62 | Rectangle |
| Example 30 | 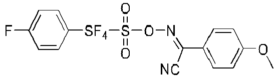 | 270.8 | 30 | 63 | Rectangle |
| Example 31 | 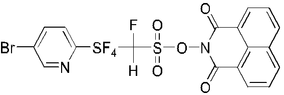 | 295.1 | 31 | 64 | Rectangle |
| Example 32 | 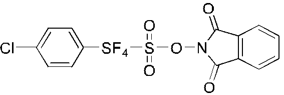 | 270 | 32 | 66 | Rectangle |

As is clear from the tables, the compound of the present invention can generate a sulfonic acid with higher acid strength and form a higher resolution pattern than the conventional acid generators described in Reference Examples and Comparative Examples.

Therefore, it is understood that a semiconductor device having a high-resolution wiring pattern or circuit pattern can be manufactured with a high yield by using the compound of the present invention.

In summary, the structure of the present disclosure and variations thereof are as appended below.
[1] A compound which is a salt of an anion represented by formula (a-1) or (a-2) and a cation.
[2] A compound represented by formula (b-1) or (b-2).
[3] A compound represented by formula (c-1) or (c-2).
[4] A compound represented by formula (d-1) or (d-2).
[5] An acid generator comprising the compound according to any one of [1] to [4].
[6] A photoresist comprising the acid generator according to [5] and an acid-reactive compound.
[7] A method for manufacturing an electronic device or an optical device, comprising the step of forming a pattern by photolithography using the photoresist according to [6].
[8] Use of the compound according to any one of [1] to [4] as an acid generator.
[9] A photoresist comprising the compound according to any one of [1] to [4] and an acid-reactive compound.
[10] Use of a composition comprising the compound according to any one of [1] to [4] and an acid-reactive compound as a photoresist.
[11] A compound which is a salt of an anion represented by formula (a-1) or (a-2) and a sulfonium ion or an iodonium ion.
[12] A compound which is a salt of an anion represented by formula (a-1) or (a-2) and an arylsulfonium ion or an aryliodonium ion.
[13] A compound which is a salt of an anion represented by formula (a-1) or (a-2) and a sulfonium ion represented by formula (s).

### Industrial Applicability

The compound of the present invention has no perfluoromethyl group or perfluoromethylene group and therefore falls outside PFAS regulations. The compound easily decomposes under light irradiation to generate a sulfonic acid with high acid strength.

A resist film having a high-resolution pattern with good accuracy can be manufactured by using a photoresist containing the compound of the present invention. When a substrate is subjected to an etching process using the resist film obtained, a semiconductor device having a high-resolution pattern can be manufactured with a high yield.

## Claims

1. A compound which is a salt of an anion represented by formula (a-1) or (a-2): 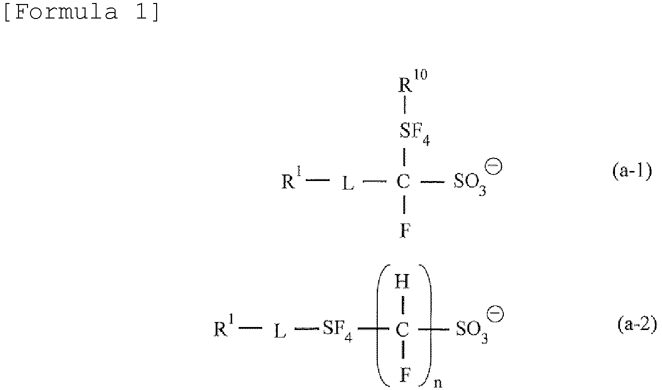 and a cation, wherein R¹ represents an optionally substituted hydrocarbon group or heterocyclic group; L represents a single bond or a linking group; and R¹⁰ represents a fluorine atom, an aromatic hydrocarbon group, or an aromatic heterocyclic group, and n represents 0 or 1.

2. A compound represented by formula (b-1) or (b-2) : 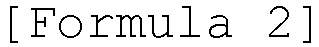 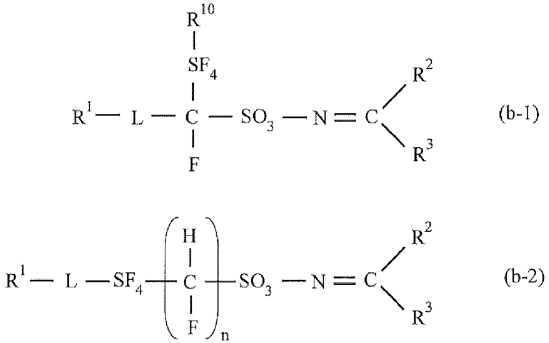 wherein R¹ represents an optionally substituted hydrocarbon group or heterocyclic group; L represents a single bond or a linking group; R² and R³ are the same as or different from each other and each represent an organic group; and R¹⁰ represents a fluorine atom, an aromatic hydrocarbon group, or an aromatic heterocyclic group, and n represents 0 or 1.

3. A compound represented by formula (c-1) or (c-2) : 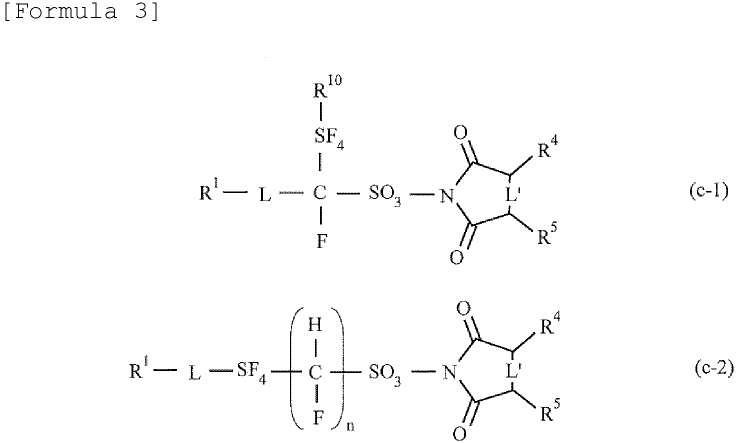 wherein R¹ represents an optionally substituted hydrocarbon group or heterocyclic group; L and L' are the same as or different from each other and each represents a single bond or a linking group; R⁴ and R⁵ are the same as or different from each other and each represent an optionally substituted hydrocarbon group; R⁴ and R⁵ are optionally linked to each other to form a ring together with an adjacent carbon atom; and R¹⁰ represents a fluorine atom, an aromatic hydrocarbon group, or an aromatic heterocyclic group, and n represents 0 or 1.

4. A compound represented by formula (d-1) or (d-2) : 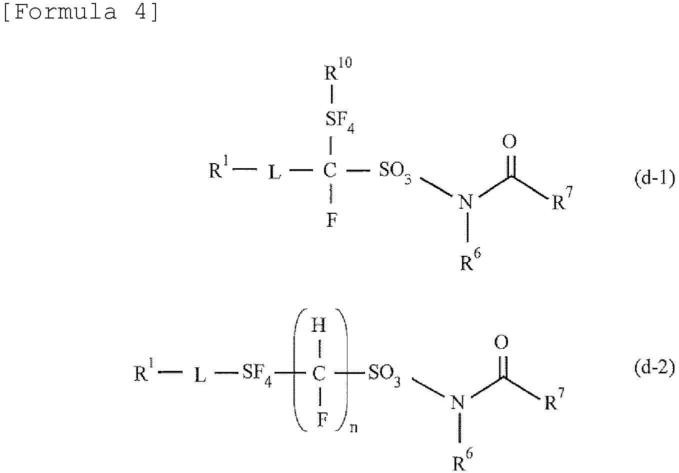 wherein R¹ represents an optionally substituted hydrocarbon group or heterocyclic group; L represents a single bond or a linking group; R⁶ and R⁷ are the same as or different from each other and each represent an optionally substituted hydrocarbon group; and R¹⁰ represents a fluorine atom, an aromatic hydrocarbon group, or an aromatic heterocyclic group, and n represents 0 or 1.

5. An acid generator comprising the compound according to any one of claims 1 to 4.

6. A photoresist comprising the acid generator according to claim 5 and an acid-reactive compound.

7. A method for manufacturing an electronic device or an optical device, comprising the step of forming a pattern by photolithography using the photoresist according to claim 6.
